# EUROPEAN PATENT APPLICATION

(11) **EP 4 276 172 A1**
(43) Date of publication of application: **15.11.2023**
(21) Application number: 22736784.4
(22) Date of filing: 11.01.2022
(51) Int. Cl.: C12N 5/077, C12N 5/10

(54) **MEDIUM FOR CULTURING AND EXPANDING NEPHRON PROGENITOR CELLS, METHOD FOR CULTURING AND EXPANDING NEPHRON PROGENITOR CELLS, AND METHOD FOR PRODUCING RENAL ORGANOIDS**

(30) Priority: 08.01.2021 JP 2021002203
(71) Applicant: Kyoto University, Kyoto-shi, Kyoto 606-8501 (JP); Rege Nephro Co., Ltd., Kyoto-shi, Kyoto, 606-8501 (JP)
(72) Inventor: OSAFUNE, Kenji, Kyoto-shi, Kyoto 606-8501 (JP); ARAOKA, Toshikazu, Kyoto-shi, Kyoto 606-8501 (JP); KOBAYASHI, Yoshifumi, Kyoto-shi, Kyoto 606-8501 (JP); RYOSAKA, Makoto, Kyoto-shi, Kyoto 606-8501 (JP); TSUJIMOTO, Hiraku, Kyoto-shi, Kyoto 606-8501 (JP)
(74) Representative: Cabinet Becker et Associés
(86) International application number: PCT/JP2022/000564
(87) International publication number: WO 2022/149616

(57) **Abstract**

A medium for culturing and expanding nephron progenitor cells, the medium containing a GSK-3β inhibitor, a ROCK inhibitor, and at least one fibroblast growth factor selected from the group consisting of FGF9 and FGF20. Also, a method for culturing and expanding nephron progenitor cells using the medium. Also, a method for producing renal organoids, the method including a step of culturing and expanding nephron progenitor cells using the above method for culturing and expanding nephron progenitor cells, and a step of differentiating the cultured and expanded nephron progenitor cells into renal organoids.

## Description

### [Technical Field]

The present invention relates to a medium for culturing and expanding nephron progenitor cells, a method for culturing and expanding nephron progenitor cells, and a method for producing renal organoids.

Priority is claimed on Japanese Patent Application No. 2021-002203, filed January 8, 2021, the content of which is incorporated herein by reference.

The number of patients in Japan currently suffering from chronic kidney disease (CKD) is estimated at about 13 million, and it is now being called a new national disease. There are few radical treatments for chronic kidney disease, and the number of End-stage renal disease patients requiring dialysis due to progression of the disease exceeds 340,000 in Japan, which is a significant problem not only medically, but also in terms of the economic costs of treatment. Kidney transplantation is one example of a radical treatment for chronic kidney disease including End-stage renal disease, but due to a severe shortage of donor kidneys, the supply is a long way from meeting the demand.

Kidneys are derived from the early embryonic tissue known as the intermediate mesoderm, and in vertebrates, three kidneys comprising the pronephros, the mesonephros and the metanephros are formed from the intermediate mesoderm, whereas in mammals, the metanephros forms the mature kidney. The metanephros is generated by interaction between two tissue types: the tissue known as the mesenchyme that differentiates into the nephrons and stroma of the mature kidney, and the tissue known as the ureteric bud that differentiates into the renal pelvis, the ureter and a portion of the bladder located distally from the collecting ducts of the mature kidney. Moreover, the existence in the metanephric mesenchyme of nephron progenitor cells (NPC) which exhibit pluripotency and differentiate into the glomerulus and a plurality of types of tubular epithelial cells that constitute the nephrons has already been disclosed (Non-Patent Documents 1 and 2).

One method that has been reported for culturing and expanding mouse nephron progenitor cells (mNPC) is a method that uses bone morphogenetic protein (BMP) 7. For example, Non-Patent Document 3 reports that by using a medium (NPSR medium) containing BMP7, fibroblast growth factor (FGF) 2, heparin, Y-27632, CHIR99021, leukemia inhibitory factor (LIF), A 83-01 and LDN193189, mNPC could be grown across a period of one year or longer.

### [Citation List]

### [Non-Patent Documents]

[Non-Patent Document 1]
   Osafune K., et al., Identification of multipotent progenitors in the embryonic mouse kidney by a novel colony-forming assay. Development 2006; 133: 151 to 61.
[Non-Patent Document 2]
   Kobayashi A., et al., Six2 defines and regulates a multipotent self-renewing nephron progenitor population throughout mammalian kidney development. Cell Stem Cell 2008; 3: 169 to 81.
[Non-Patent Document 3]
   Z. Li, T. Araoka, J.C.I. Belmonte, Gene Editing in 3D Cultured Nephron Progenitor Cell Lines, in: S. Vainio (Ed.), Kidney Organog Methods Protoc, Hummana Press, New York, 2019: pp. 151 to 159.

### [Summary of Invention]

### [Technical Problem]

In order to enable cultured and expanded nephron progenitor cells to be utilized in regenerative medicine and the like, it is necessary to retain the characteristics of the nephron progenitor cells such as differentiation potency during the culturing and expansion process. Consequently, the development of a culturing method that enables efficient culturing and expansion of nephron progenitor cells while retaining the characteristics of the nephron progenitor cells would be very desirable.

Accordingly, the present invention has the objects of providing a medium for culturing and expanding nephron progenitor cells that enables culturing and expansion of the nephron progenitor cells with good retention of the differentiation potency, a method for culturing and expanding nephron progenitor cells using the medium, and a method for producing renal organoids from nephron progenitor cells obtained using the culturing and expansion method.

### [Solution to Problem]

The present invention includes the following aspects.
[1] A medium for culturing and expanding nephron progenitor cells, the medium containing a GSK-3β inhibitor, a ROCK inhibitor, and at least one fibroblast growth factor selected from the group consisting of FGF9 and FGF20.
[2] The medium for culturing and expanding nephron progenitor cells according to [1], also containing a JAK inhibitor.
[3] The medium according to [2], wherein the JAK inhibitor is a JAK2 inhibitor.
[4] The medium according to [3], wherein the JAK2 inhibitor is TG101348.
[5] The medium according to any one of [1] to [4], wherein the GSK-3β inhibitor is CHIR99021.
[6] The medium according to any one of [1] to [5], wherein the ROCK inhibitor is Y-27632.
[7] The medium according to any one of [1] to [6], also containing 10% by volume of AS401 relative to the total volume of the medium.
[8] The medium according to any one of [1] to [7], wherein the nephron progenitor cells are human nephron progenitor cells.
[9] The medium according to any one of [1] to [8], wherein the nephron progenitor cells are nephron progenitor cells induced from pluripotent stem cells.
[10] The medium according to [9], wherein the pluripotent stem cells are iPS cells.
[11] A method for culturing and expanding nephron progenitor cells, the method including a step of culturing nephron progenitor cells in the medium according to any one of [1] to [10].
[12] The method for culturing and expanding nephron progenitor cells according to [11], wherein the step of culturing nephron progenitor cells includes subculturing of the nephron progenitor cells.
[13] A method for culturing and expanding nephron progenitor cells, the method including (A) a step of culturing nephron progenitor cells in the medium according to any one of [1] to [10] for 30 to 60 hours, and (B) a step of culturing the cells obtained in the step (A) in a medium containing a GSK-3β inhibitor and at least one fibroblast growth factor selected from the group consisting of FGF9 and FGF20, but containing no ROCK inhibitor.
[14] The method for culturing and expanding nephron progenitor cells according to [13], the method further including (C) a step of subculturing the cells obtained in the step (B) in the medium according to any one of [1] to [10].
[15] The method for culturing and expanding nephron progenitor cells according to [14], the method further including (D) a step of culturing the cells subcultured in the step (C) in the medium according to any one of [1] to [10] for 30 to 60 hours, and (E) a step of culturing the cells obtained in the step (D) in a medium containing a GSK-3β inhibitor and at least one fibroblast growth factor selected from the group consisting of FGF9 and FGF20, but containing no ROCK inhibitor.
[16] The method for culturing and expanding nephron progenitor cells according to any one of [11] to [15], wherein the nephron progenitor cells are human nephron progenitor cells.
[17] The method for culturing and expanding nephron progenitor cells according to any one of [11] to [16], wherein the nephron progenitor cells are nephron progenitor cells induced from pluripotent stem cells.
[18] The method for culturing and expanding nephron progenitor cells according to [17], wherein the pluripotent stem cells are iPS cells.
[19] The method for culturing and expanding nephron progenitor cells according to [17] or [18], wherein the nephron progenitor cells are nephron progenitor cells obtained using a method including steps (i) to (vi) below: (i) a step of culturing pluripotent stem cells in a medium containing FGF2, BMP4, a GSK-3β inhibitor, and retinoic acid or a derivative thereof; (ii) a step of culturing the cells obtained in the step (i) in a medium containing FGF2, a GSK-3β inhibitor and BMP7; (iii) a step of culturing the cells obtained in the step (ii) in a medium containing a GSK-3β inhibitor, BMP7 and a TGFβ inhibitor, but containing no FGF2; (iv) a step of culturing the cells obtained in the step (iii) in a medium containing FGF2, a GSK-3β inhibitor, activin and a ROCK inhibitor; (v) a step of culturing the cells obtained in the step (iv) in a medium containing retinoic acid or a derivative thereof, but containing no FGF9; and (vi) a step of culturing the cells obtained in the step (v) in a medium containing a GSK-3β inhibitor and at least one fibroblast growth factor selected from the group consisting of FGF9 and FGF20.
[20] The method for culturing and expanding nephron progenitor cells according to [19], wherein the medium used in the step (iv) contains no BMP7.
[21] The method for culturing and expanding nephron progenitor cells according to [19] or [20], wherein at least one of the steps (i) to (vi) is conducted using a culture vessel having a cell contact area of at least 400 cm².
[22] A method for producing renal organoids, the method including a step of culturing and expanding nephron progenitor cells using the method for culturing and expanding nephron progenitor cells according to any one of [11] to [21], and a step of differentiating the cultured and expanded nephron progenitor cells into renal organoids.

### [Advantageous Effects of Invention]

The present invention is able to provide a medium for culturing and expanding nephron progenitor cells that enables culturing and expansion of the nephron progenitor cells with good retention of the differentiation potency, a method for culturing and expanding nephron progenitor cells using the medium, and a method for producing renal organoids from nephron progenitor cells obtained using the culturing and expansion method.

### [Brief Description of Drawings]

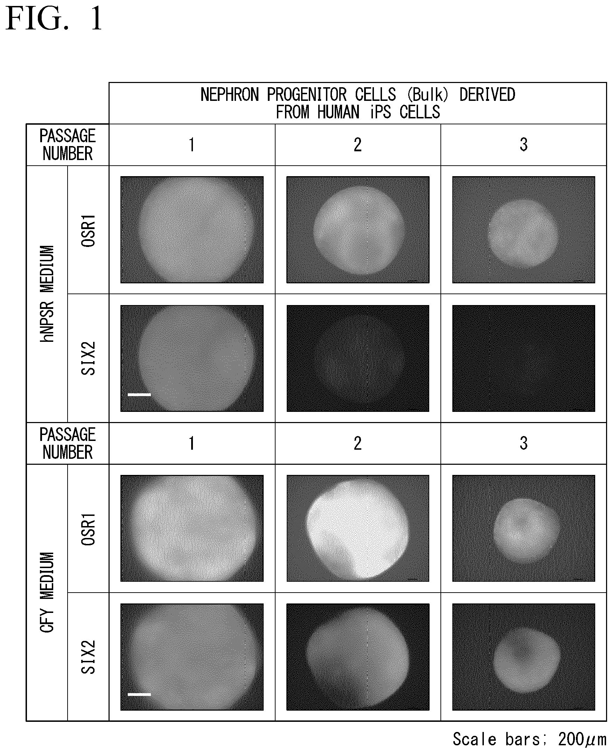

FIG. 1 illustrates the results of subculturing hNPC (Bulk) using either an hNPSR medium (see Table 1) or a CFY medium (see Table 2), and confirming the expression of S1X2 and OSR1 in each subculture. The hNPC used were differentially induced from hiPSC using a differentiation induction method A.

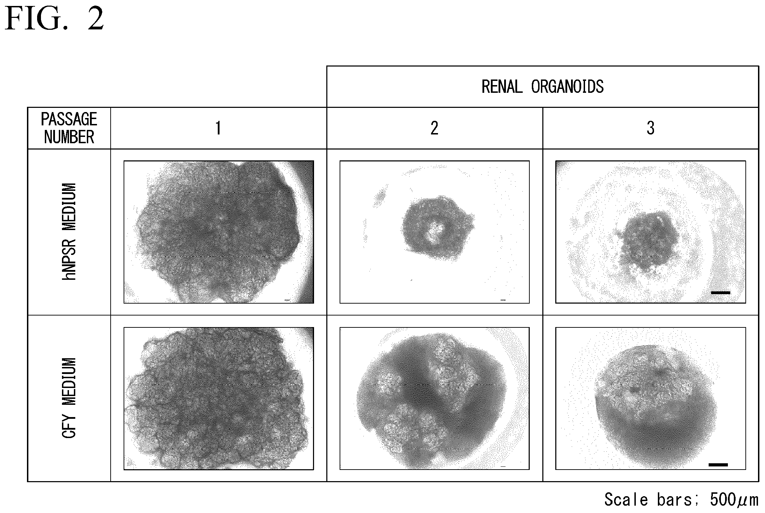

FIG. 2 illustrates the results of testing the production of renal organoids from hNPC (Bulk) that had been subcultured using either the hNPSR medium or the CFY medium. The hNPC used were differentially induced from hiPSC using the differentiation induction method A.

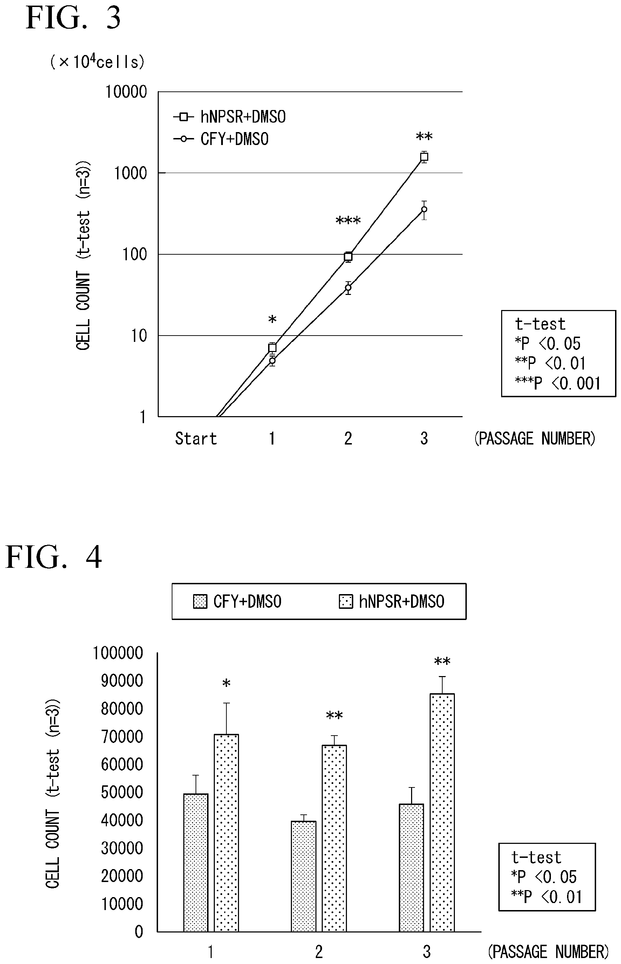

FIG. 3 illustrates the results of subculturing hNPC (Bulk) using a medium prepared by adding DMSO to either the hNPSR medium or the CFY medium, measuring the cell count and then calculating the cumulative cell count. The hNPC used were differentially induced from hiPSC using the differentiation induction method A.
FIG. 4 illustrates the results of subculturing hNPC (Bulk) using a medium prepared by adding DMSO to either the hNPSR medium or the CFY medium, and measuring the cell count in each passage. The hNPC used were differentially induced from hiPSC using the differentiation induction method A.

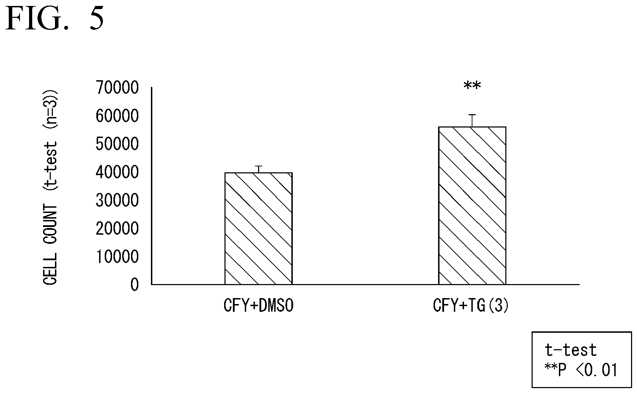

FIG. 5 illustrates the results of culturing hNPC (Bulk) using either a medium prepared by adding DMSO to the CFY medium (CFY+DMSO) or a medium prepared by adding 3nM TG101348 to the CFY medium (CFT+TG(3)), and measuring the cell count. The hNPC used were differentially induced from hiPSC using the differentiation induction method A.

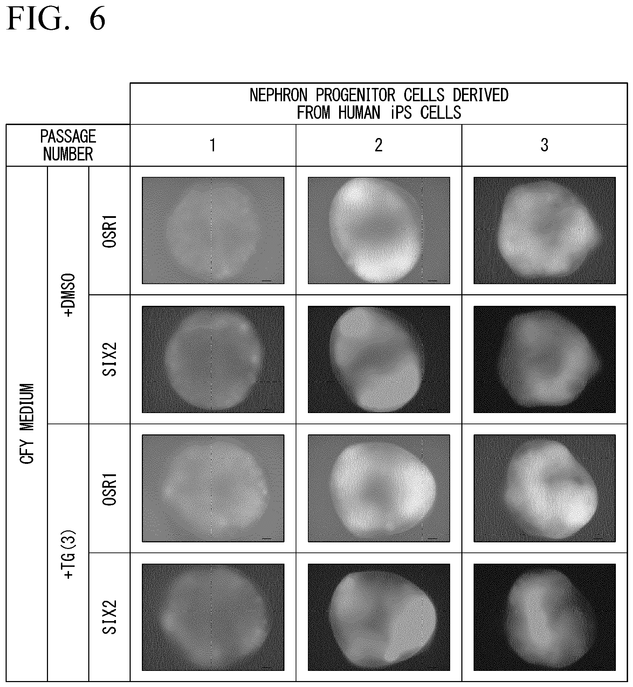

FIG. 6 illustrates the results of subculturing hNPC (Bulk) using either a medium prepared by adding DMSO to the CFY medium (CFY+DMSO) or a medium prepared by adding 3nM TG101348 to the CFY medium (CFT+TG(3)), and confirming the expression of SIX2 and OSR1 in each passage. The hNPC used were differentially induced from hiPSC using the differentiation induction method A.

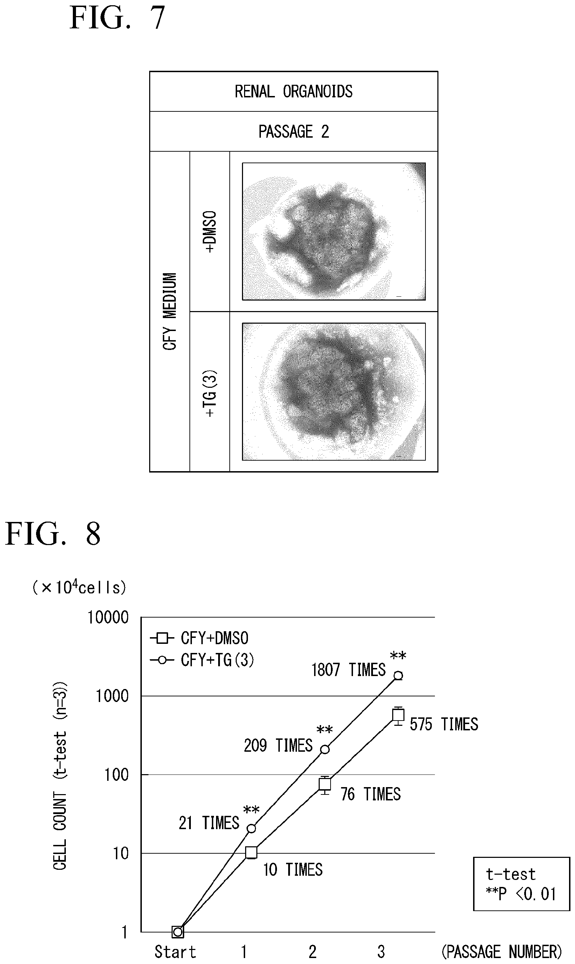

FIG. 7 illustrates the results of subculturing hNPC (Bulk) using either a medium prepared by adding DMSO to the CFY medium (CFY+DMSO) or a medium prepared by adding 3nM TG101348 to the CFY medium (CFT+TG(3)), and testing the production of renal organoids from the cells of passage 2. The hNPC used were differentially induced from hiPSC using the differentiation induction method A.
FIG. 8 illustrates the results of culturing hNPC (c-MET(+)) using either a medium prepared by adding DMSO to the CFY medium (CFY+DMSO) or a medium prepared by adding 3nM TG101348 to the CFY medium (CFT+TG(3)), measuring the cell count in each passage, and then calculating the cumulative cell count. The hNPC used were differentially induced from hiPSC using the differentiation induction method A.

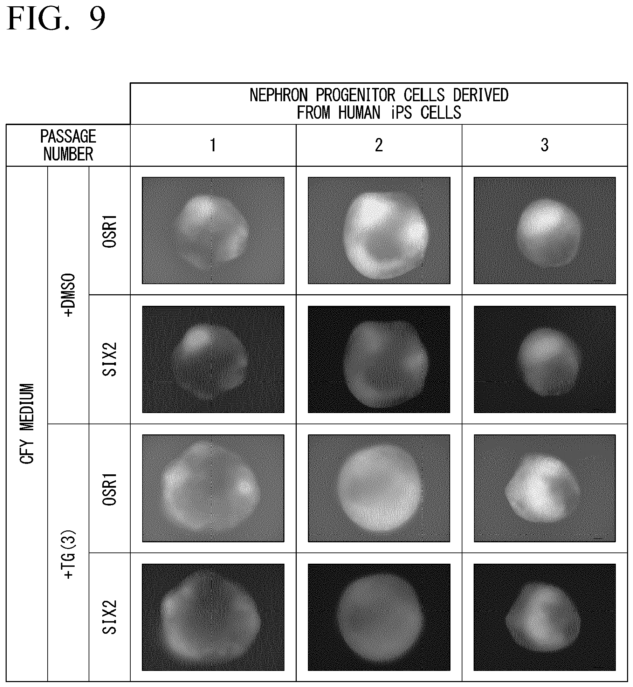

FIG. 9 illustrates the results of subculturing hNPC (c-MET(+)) using either a medium prepared by adding DMSO to the CFY medium (CFY+DMSO) or a medium prepared by adding 3nM TG101348 to the CFY medium (CFT+TG(3)), and confirming the expression of SIX2 and OSR1 in each passage. The hNPC used were differentially induced from hiPSC using the differentiation induction method A.

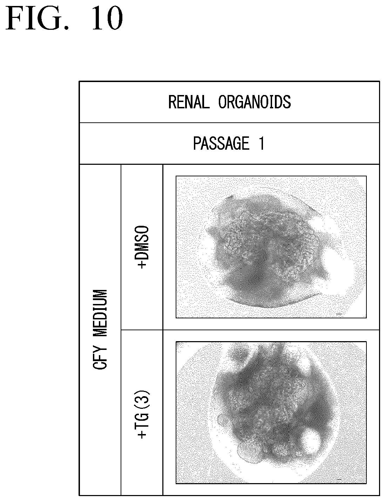

FIG. 10 illustrates the results of subculturing hNPC (c-MET(+)) using either a medium prepared by adding DMSO to the CFY medium (CFY+DMSO) or a medium prepared by adding 3nM TG101348 to the CFY medium (CFT+TG(3)), and testing the production of renal organoids from the cells of passage 1. The hNPC used were differentially induced from hiPSC using the differentiation induction method A.

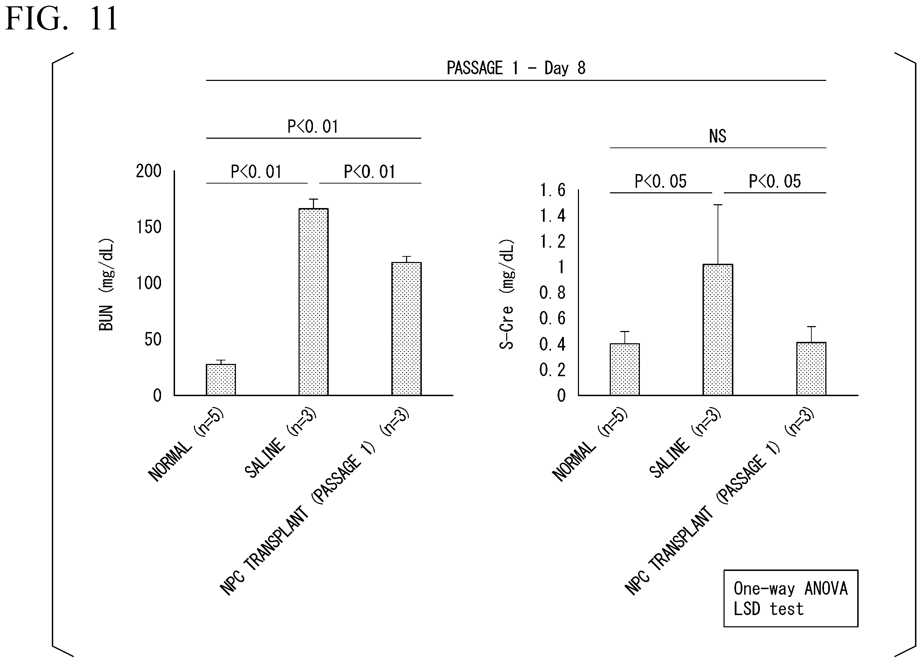

FIG. 11 illustrates the results of transplanting a cell mass of hNPC (Bulk) from one passage in the CFY medium beneath the renal capsule of an immunodeficient mouse that had been administered with cisplatin to cause the onset of acute kidney injury (AKI) (namely, an AKI mouse), and then measuring the levels of blood urea nitrogen (BUN) and serum creatine (S-Cre). In the figure, "Normal" indicates a normal mouse, "Saline" indicates an AKI mouse that had been administered with physiological saline beneath the renal capsule, and "NPC transplant" indicates the AKI mouse subjected to the hNPC (Bulk) transplant. The hNPC used were differentially induced from hiPSC using the differentiation induction method A.

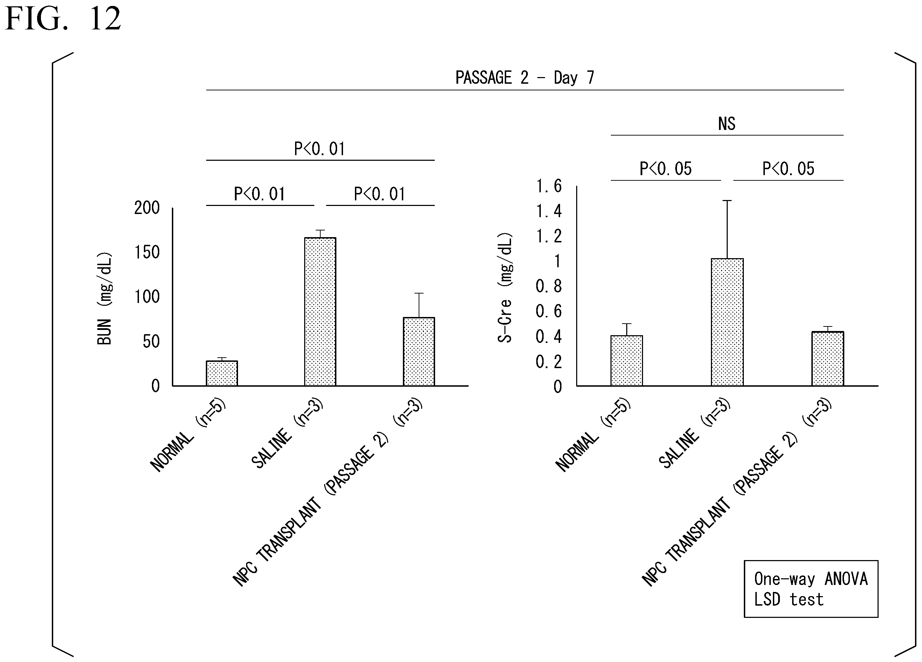

FIG. 12 illustrates the results of transplanting a cell mass of hNPC (Bulk) from passage 2 in the CFY medium beneath the renal capsule of an immunodeficient mouse that had been administered with cisplatin to cause the onset of acute kidney injury (AKI), and then measuring the levels of blood urea nitrogen (BUN) and serum creatine (S-Cre). In the figure, "Normal" indicates a normal mouse, "Saline" indicates an AKI mouse that had been administered with physiological saline beneath the renal capsule, and "NPC transplant" indicates the AKI mouse subjected to the hNPC (Bulk) transplant. The hNPC used were differentially induced from hiPSC using the differentiation induction method A.

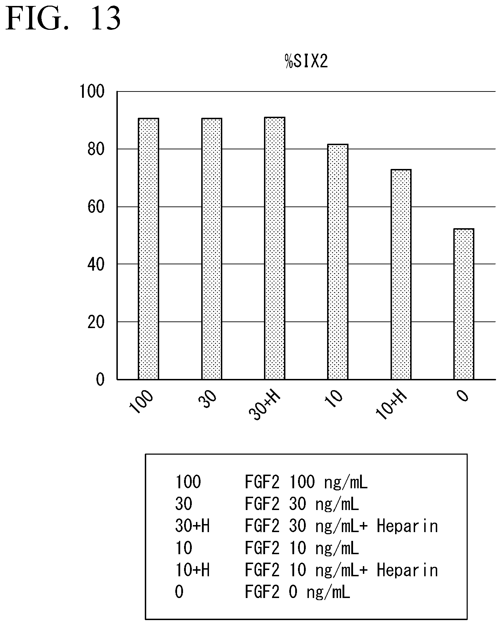

FIG. 13 illustrates the results of confirming, in the induction of hNPC from human iPS cells (hiPSC) using a differentiation induction method B (see Example 5), the differentiation induction efficiency of SIX2-positive cells when the FGF2 concentration was varied, and when heparin was added, in step 4 (production of the metanephric-lineage late primitive streak).

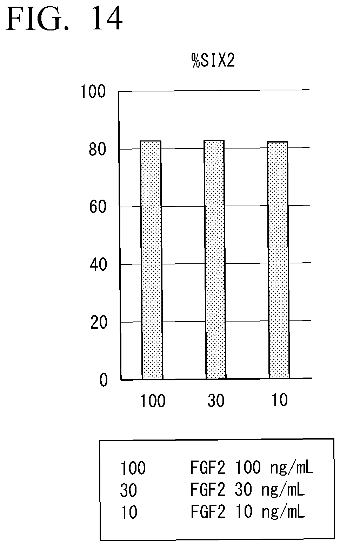

FIG. 14 illustrates the results of confirming, in the induction of hNPC from hiPSC using the differentiation induction method B, the differentiation induction efficiency of SIX2-positive cells when the FGF2 concentration was varied in step 3 (production of the mesodermal-lineage late primitive streak).

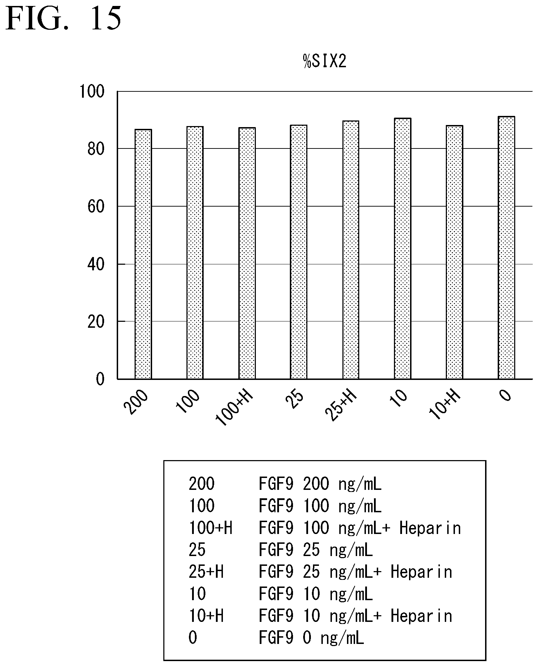

FIG. 15 illustrates the results of confirming, in the induction of hNPC from hiPSC using the differentiation induction method B, the differentiation induction efficiency of SIX2-positive cells when the FGF9 concentration was varied, and when heparin was added, in step 5 (production of the late posterior intermediate mesoderm).

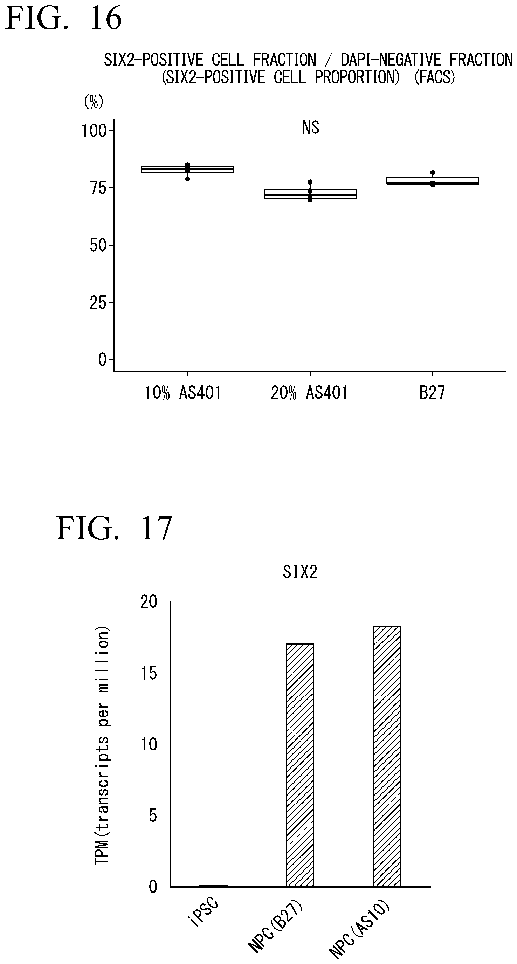

FIG. 16 illustrates the results of confirming, in the induction of hNPC from hiPSC using the differentiation induction method B, the differentiation induction efficiency of SIX2-positive cells when the additive of the basal medium was changed from B27 (B27 Supplement (50×) minus vitamin A, Invitrogen Corporation) to AS401 (StemFit (a registered trademark) for Differentiation, Ajinomoto Healthy Supply Co., Inc.). 10% AS401: a basal medium containing 10% by volume of AS401 relative to the total volume of the basal medium; 20% AS401: a basal medium containing 20% by volume of AS401 relative to the total volume of the basal medium; B27: a basal medium containing added B27. Hereinafter, the term "medium containing 10% added AS401" means a medium containing 10% by volume of AS401 relative to the total volume of the basal medium. The term "medium containing 20% added AS401" has a similar meaning.
FIG. 17 illustrates the results of confirming SIX2 expression when the basal medium additive was changed from B27 to AS401 in the induction of hNPC from clinical hiPSC using the differentiation induction method B. iPSC: clinical hiPSC; NPC (B27): NPC induced using a basal medium containing added B27; NPC(AS10): hNPC induced using a basal medium containing 10% added AS401.

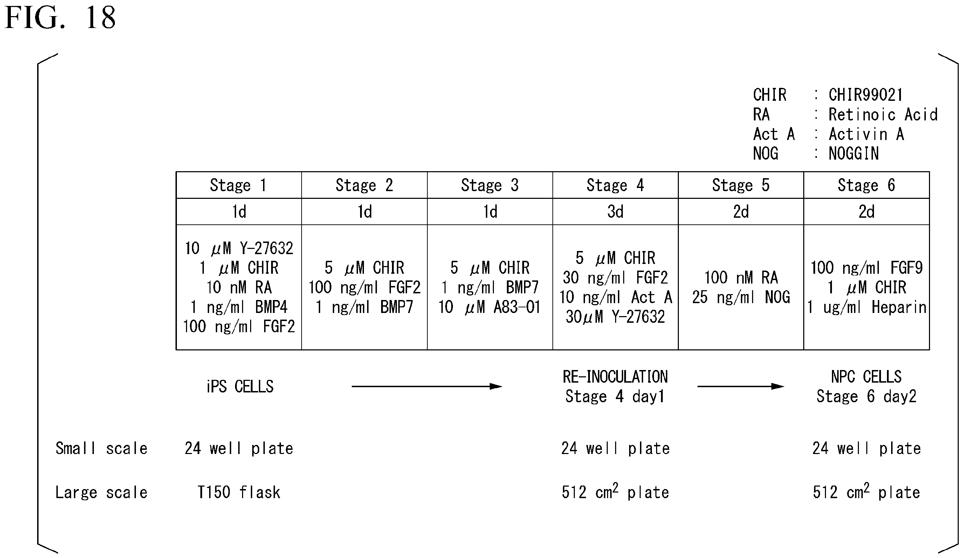

FIG. 18 indicates the differentiation induction conditions for inducing hNPC from hiPSC in Examples 10 to 14 (differentiation induction method C). Hereinafter, the small scale differentiation induction method of FIG. 18 is also referred to as "differentiation induction method C (Small)", and the large scale differentiation induction method is also referred to as "differentiation induction method C (Large)". In the differentiation induction method C, unless stated otherwise, DMEM/F12 Glutamax (Thermo Fischer Scientific Inc.) containing 10% added AS401 was used as the basal medium.

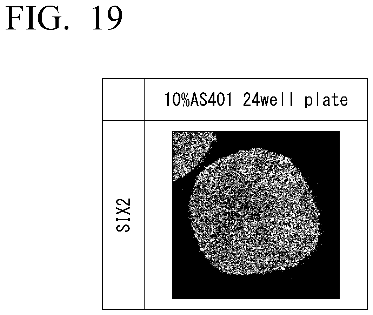

FIG. 19 illustrates a fluorescent image confirming, by immunostaining, SIX2 expression in hNPC induced from an HLA homostock hiPSC line (Ff14s04) using the differentiation induction method C (Small).

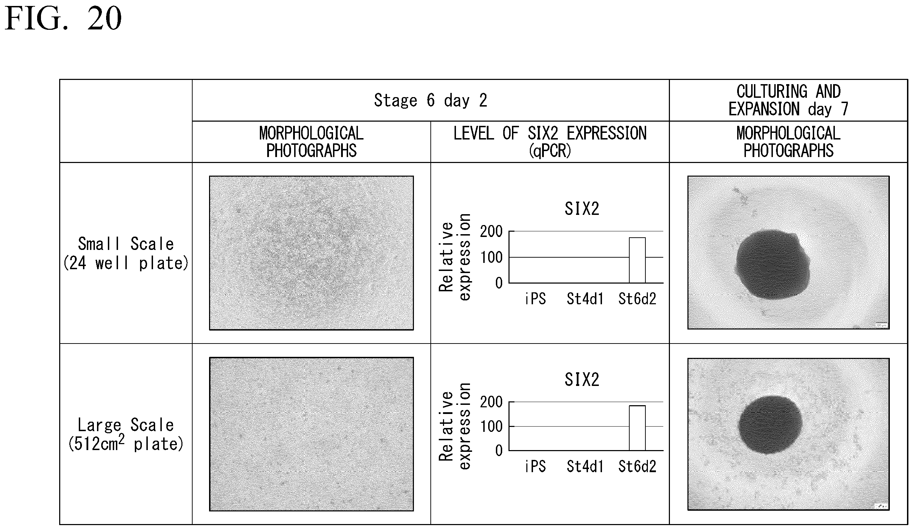

FIG. 20 illustrates the morphology and SIX2 expression on day 2 of stage 6 for hNPC differentially induced using either the differentiation induction method C (Small) or the differentiation induction method C (Large), and also illustrates a morphological photograph on day 7 following culturing and expansion.

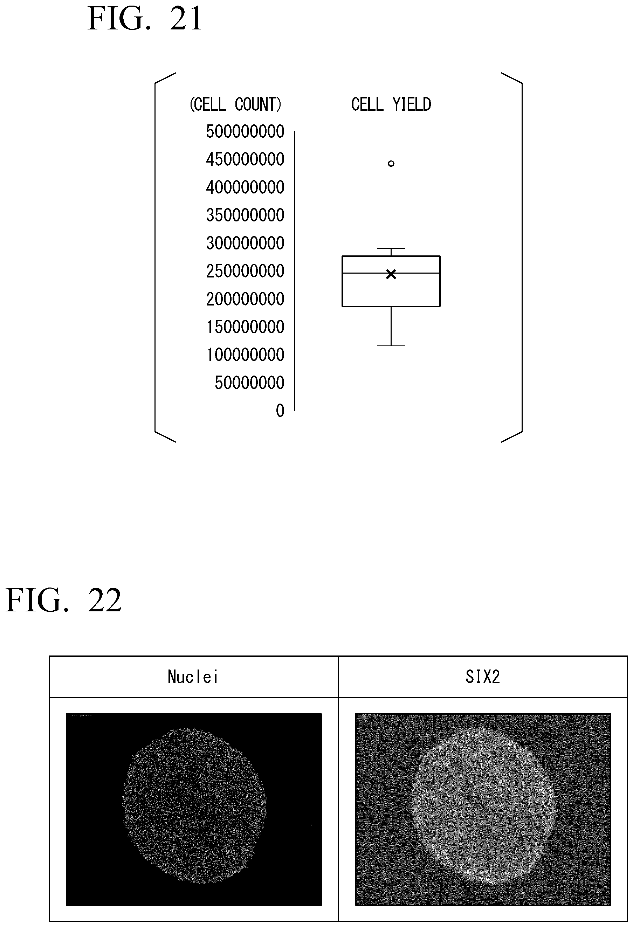

FIG. 21 indicates the cell yield of hNPC differentially induced using the differentiation induction method C (Large).
FIG. 22 illustrates a fluorescent image confirming, by immunostaining, SIX2 expression in hNPC differentially induced using the differentiation induction method C (Large).

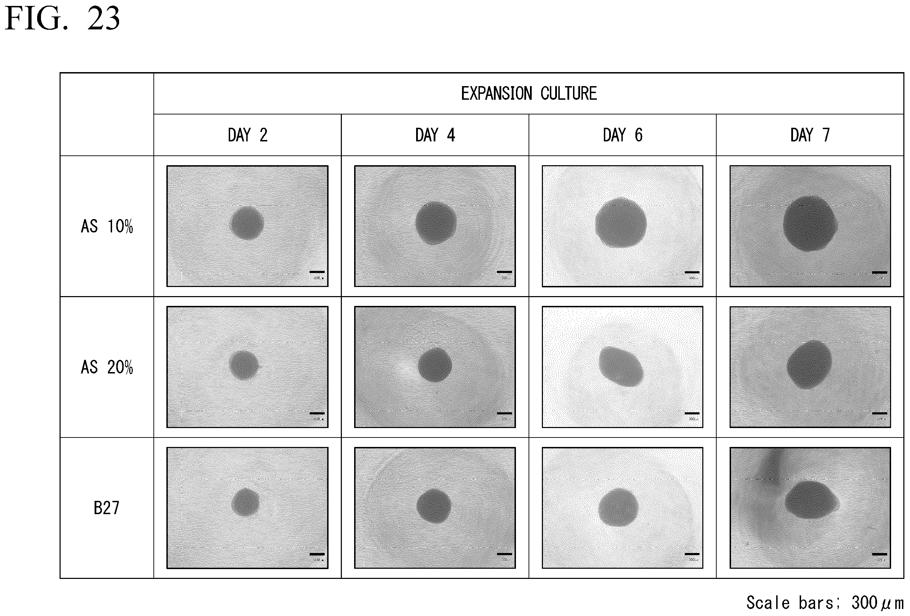

FIG. 23 illustrates morphological photographs of hNPC during culturing and expansion in media containing different additives (B27 or AS401). The scale bars represent 300 µm. AS 10%: a modified CFY medium containing 10% added AS401 instead of B27 (hereinafter also referred to as "CFY medium (-B27, +10% AS401); AS 20%: a modified CFY medium containing 20% added AS401 instead of B27 (hereinafter also referred to as "CFY medium (-B27, +20% AS401); B27: the CFY medium.

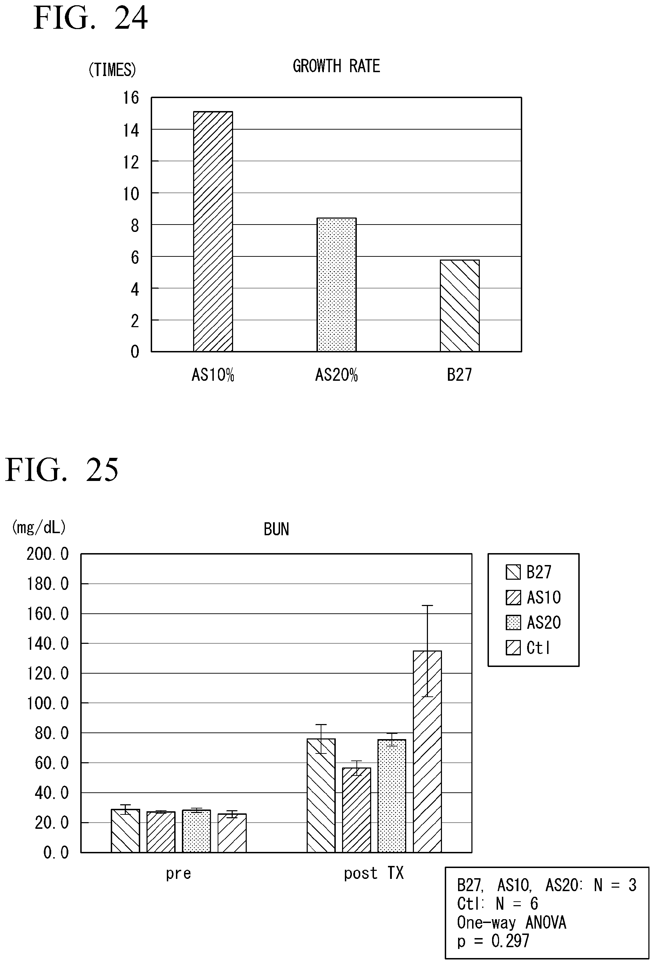

FIG. 24 illustrates the growth factors of hNPC that have been expanded and cultured in media containing different additives (B27 or AS401). AS10%: CFY medium (-B27, +10% AS401); AS20%: CFY medium (-B27, +20% AS401); B27: the CFY medium.
FIG. 25 illustrates the results of transplanting cell masses of hNPC expanded and cultured in media containing different additives (B27 or AS401) beneath the renal capsule of AKI mice, and then measuring the levels of blood urea nitrogen (BUN). B27: transplantation of cell mass ofhNPC expanded and cultured in CFY medium; AS10: transplantation of cell mass of hNPC expanded and cultured in CFY medium (-B27, +10% AS401); AS20: transplantation of cell mass of hNPC expanded and cultured in CFY medium (-B27, +20% AS401); Ctl: no transplant. pre: prior to cisplatin administration; post TX: after cisplatin administration.

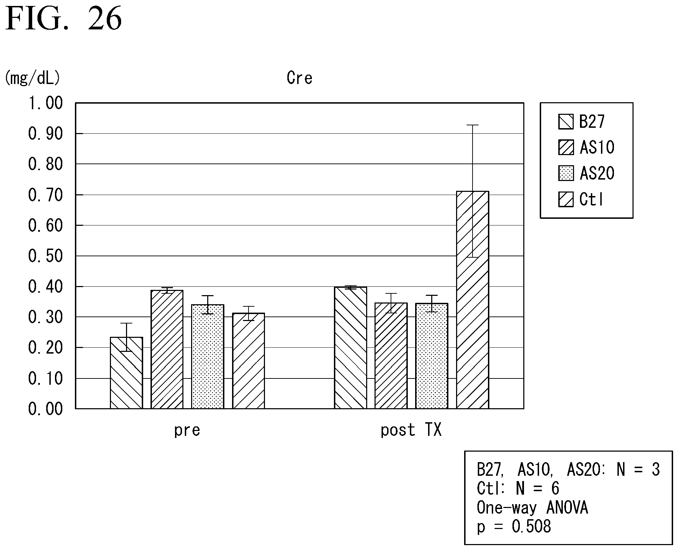

FIG. 26 illustrates the results of transplanting cell masses of hNPC expanded and cultured in media containing different additives (B27 or AS401) beneath the renal capsule of AKI mice, and then measuring the levels of serum creatine (S-Cre). B27: transplantation of cell mass of hNPC expanded and cultured in CFY medium; AS10: transplantation of cell mass of hNPC expanded and cultured in CFY medium (-B27, +10% AS401); AS20: transplantation of cell mass of hNPC expanded and cultured in CFY medium (-B27, +20% AS401); Ctl: no transplant. pre: prior to cisplatin administration; post TX: after cisplatin administration.

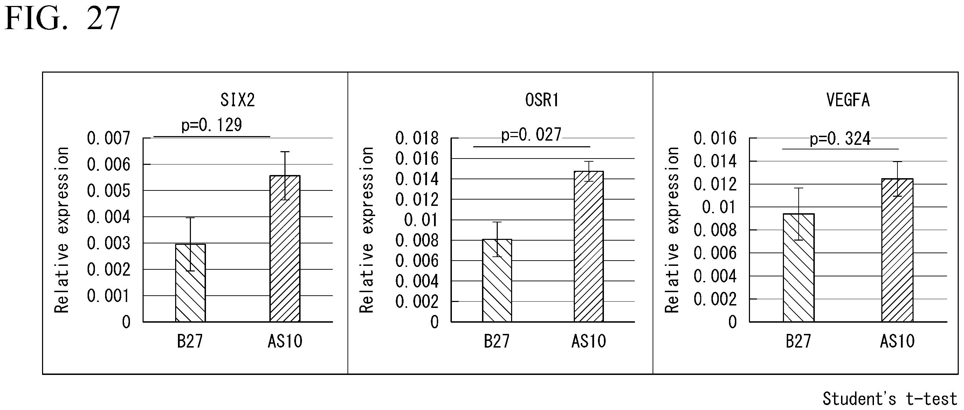

FIG. 27 illustrates results of confirming gene expression in hNPC expanded and cultured in media containing different additives (B27 or AS401). B27: the CFY medium; AS10: CFY medium (-B27, +10% AS401).

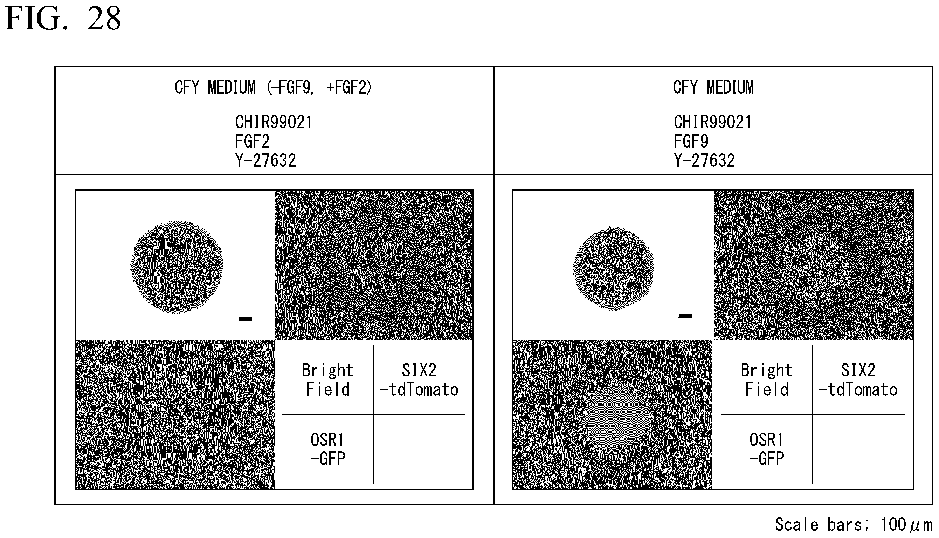

FIG. 28 illustrates the expression of NPC markers in hNPC cultured and expanded using either a medium in which the FGF9 of the CFY medium had been replaced with FGF2 (hereinafter also referred to as "CFY medium (-FGF9, +FGF2)", or the CFY medium. The hNPC used were differentially induced from hiPSC using the differentiation induction method A (the differentiation induction method disclosed in International Patent Publication No. 2018/216743).

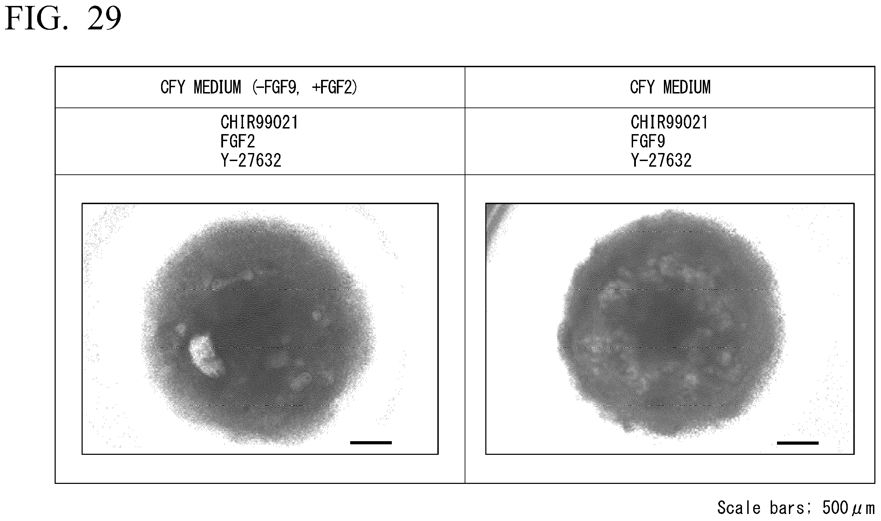

FIG. 29 illustrates the results of viewing renal organoids differentially induced from hNPC under an optical microscope. The hNPC were cultured and expanded using either the CFY medium (-FGF9, +FGF2) or the CFY medium. The hNPC used were differentially induced from hiPSC using the differentiation induction method A.

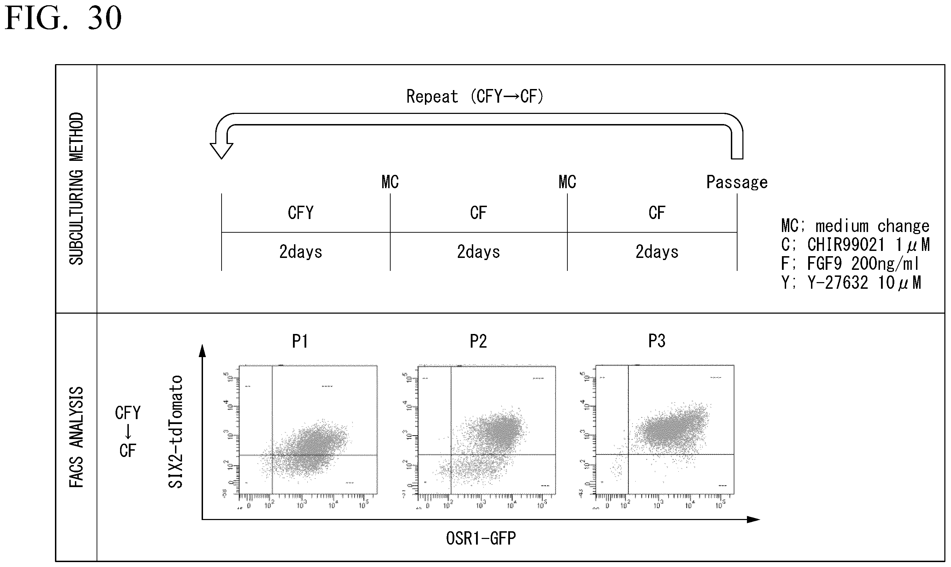

FIG. 30 illustrates the FACS analysis results of the cells of each passage when a subculturing process in which the medium was changed to a CF medium (a medium in which Y-27632 is removed from the CFY medium) two days after passage was repeated for three passages. P1: cells after passage 1; P2: cells after passage 2; P3: cells after passage 3. The hNPC used were differentially induced from hiPSC using the differentiation induction method A.

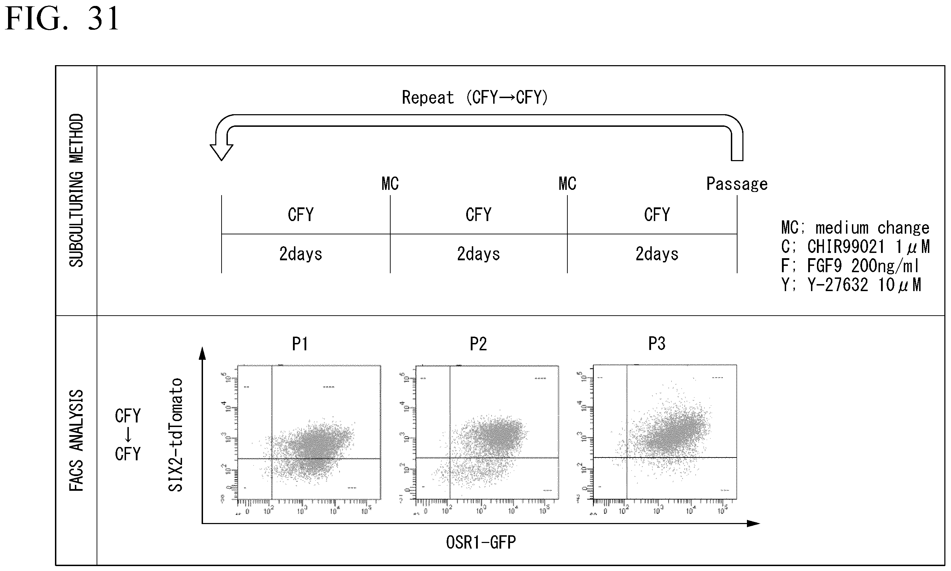

FIG. 31 illustrates the FACS analysis results of the cells of each passage when the medium change following passage was conducted with the CFY medium, and three passage repetitions were conducted. P1: cells after passage 1; P2: cells after passage 2; P3: cells after passage 3. The hNPC used were differentially induced from hiPSC using the differentiation induction method A.

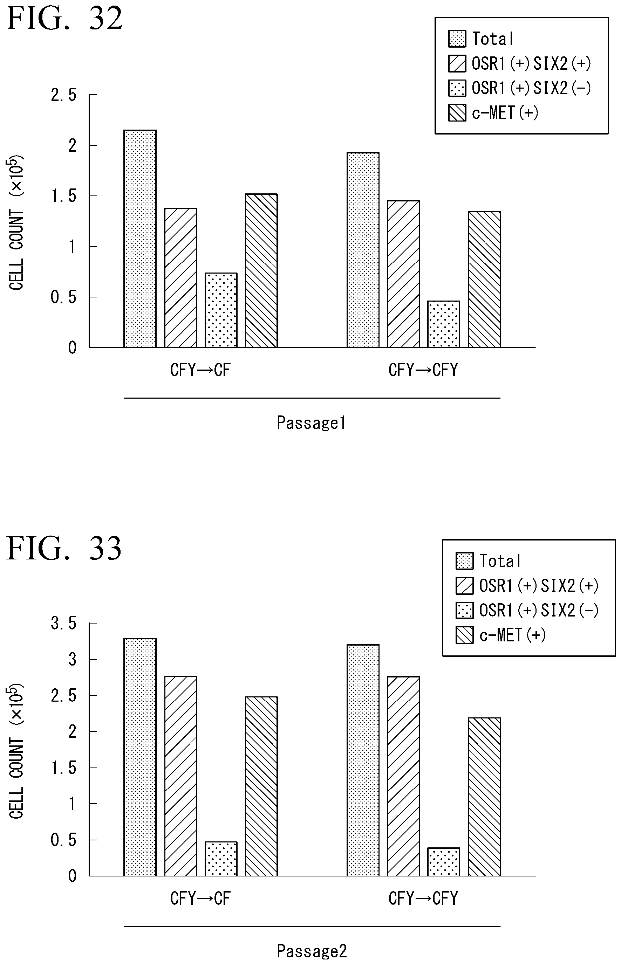

FIG. 32 illustrates the NPC marker-positive cell counts (OSR1(+)SIX2(+), OSR1(+)SIX2(-), c-MET(+)) and the total cell counts when one passage was conducted using a subculturing method in which a medium change to either the CF medium (CFY → CF) or to the CFY medium (CFY → CFY) was conducted two days after passage. The hNPC used were differentially induced from hiPSC using the differentiation induction method A.
FIG. 33 illustrates the NPC marker-positive cell counts (OSR1(+)SIX2(+), OSR1(+)SIX2(-), c-MET(+)) and the total cell counts when two passages were conducted using a subculturing method in which a medium change to either the CF medium (CFY → CF) or to the CFY medium (CFY → CFY) was conducted two days after passage. The hNPC used were differentially induced from hiPSC using the differentiation induction method A.

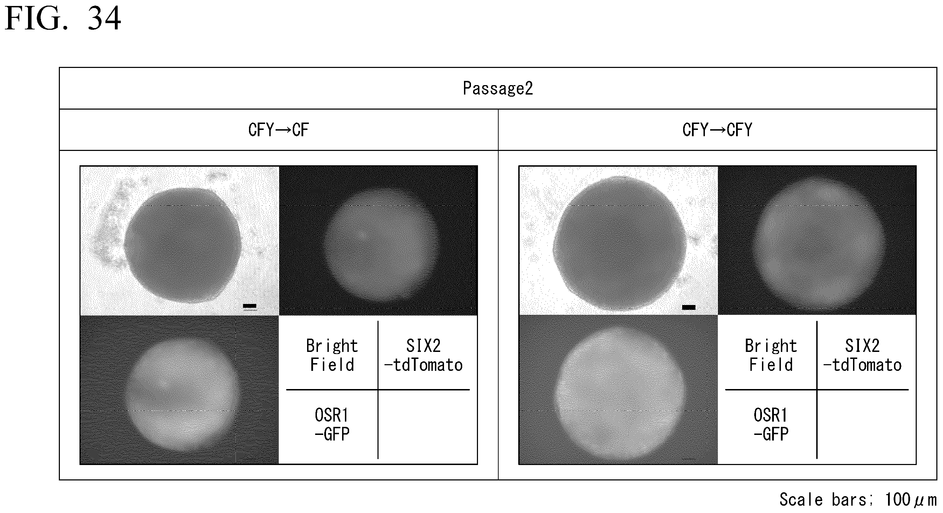

FIG. 34 illustrates NPC marker expression when two passages were conducted using a subculturing method in which a medium change to either the CF medium (CFY → CF) or to the CFY medium (CFY → CFY) was conducted two days after passage. The hNPC used were differentially induced from hiPSC using the differentiation induction method A.

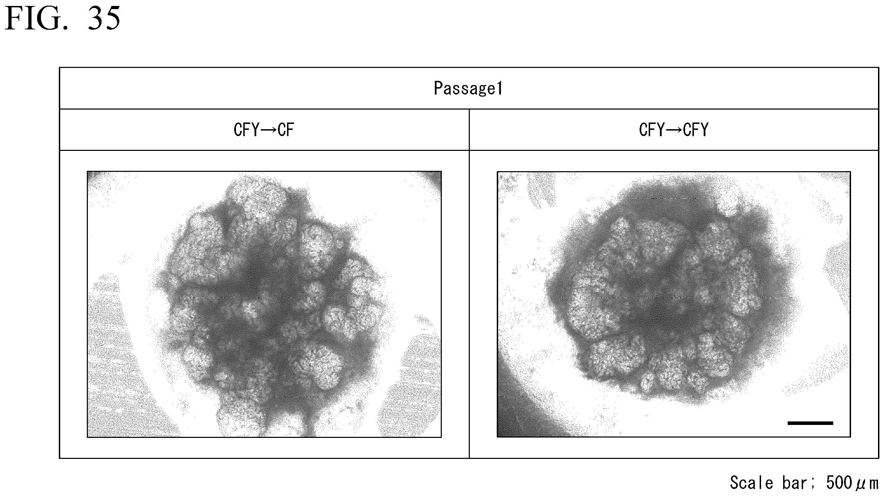

FIG. 35 illustrates the results of viewing renal organoids differentially induced from hNPC under an optical microscope. The hNPC used had undergone one passage using a subculturing method in which a medium change to either the CF medium (CFY → CF) or to the CFY medium (CFY → CFY) was conducted two days after passage. The hNPC used were differentially induced from hiPSC using the differentiation induction method A.

### [Description of Embodiments]

### <Medium for Culturing and Expanding Nephron Progenitor Cells>

A first aspect of the present invention is a medium for culturing and expanding nephron progenitor cells. The medium of this aspect contains a GSK-3β inhibitor, a ROCK inhibitor, and at least one fibroblast growth factor selected from the group consisting of FGF9 and FGF20. In one embodiment, the medium of this aspect also contains a JAK inhibitor.

### (Nephron Progenitor Cells: NPC)

NPC are cells which are capable of differentiation in vitro into organ structures such as glomerulus-like structures or renal tubule-like structures of the kidney. The differentiation potency of nephron progenitor cells into organ structures can be evaluated, for example, using a method disclosed in Osafune K, et al. (2006), Development 133: 151 to 61. SIX2 is known as a characteristics factor for maintenance of the state as a nephron progenitor cell (Cell Stem Cell 3: 169 to 181 (2008)), and examples of the nephron progenitor cells cultured using the method of the present invention include S1X2-positive nephron progenitor cells. For example, pluripotent stem cells having a reporter gene (such as tdTomato) introduced therein under the regulation of a SIX2 promoter (for example, the OSR1-GFP/SIX2-tdTomato reporter human iPS cells described in the following examples) may be cultured, and S1X2-positive nephron progenitor cells can then be isolated by a conventional method known in the technical field (such as a method using a cell sorter) using expression of the reporter gene as a marker. Further, SIX2 expression in nephron progenitor cells can also be confirmed by a gene expression analysis method such as quantitative RT-PCR (Nat Commun 4, 1367 (2013)). In this description, SIX2-positive nephron progenitor cells include cells expressing SIX2 protein, and cells expressing a protein encoded by a gene under the regulation of the SIX2 promoter. Examples of the human SIX2 gene (NCBI Gene ID: 10736) include, but are not limited to, genes having the nucleotide sequence registered under the NCBI accession number: NM_016932.5, whereas examples of the mouse SIX2 gene (NCBI Gene ID: 20472) include, but are not limited to, genes having the nucleotide sequence registered under the NCBI accession number: NM_011380.2. The nephron progenitor cells cultured in the medium of the present aspect of the invention are preferably positive for OSR1, and also positive for HOX11, WT1, SIX2 and SALL1.

There are no particular limitations on the organism that yields the nephron progenitor cells, and any animal having kidneys may be used. The nephron progenitor cells are preferably mammalian-derived cells, and more preferably human-derived cells. In other words, human nephron progenitor cells are preferred.

The nephron progenitor cells may be cells that have been isolated from the metanephric mesenchyme of an organism, or cells that have been differentially induced from pluripotent stem cells (such as ES cells or iPS cells). The nephron progenitor cells cultured and expanded in the medium of the present aspect are preferably cells that have been induced from pluripotent stem cells, and are more preferably cells induced from iPS cells.

The differential induction of nephron progenitor cells from pluripotent stem cells can be conducted using a conventional method. Examples of the method for inducing nephron progenitor cells from pluripotent stem cells include the methods disclosed in International Patent Publication No. 2014/200115, International Patent Publication No. 2017/043666, and International Patent Publication No. 2018/216743 and the like.

Isolation of the nephron progenitor cells, either from a cell population extracted from the metanephric mesenchyme, or from a cell population differentially induced from pluripotent stem cells, may be conducted, for example, using the expression of an aforementioned nephron progenitor cell marker such as OSR1, HOX11, WT1, SIX2 or SALL1 as a marker. Alternatively, the isolation may be conducted using the expression of c-MET or AGTR2 as a marker (International Patent Publication No. 2020/022261).

The nephron progenitor cells cultured and expanded in the medium of the present aspect may be provided as a cell population that also includes other cell types, or may be provided as a cell population of purified nephron progenitor cells. In the case of a cell population containing the nephron progenitor cells and one or more other cell types, the proportion of the nephron progenitor cells, relative to the total cell count (100%) is preferably 30%, at least 40%, at least 50%, at least 60%, at least 70%, at least 80%, or 90% or greater.

### (Culturing and Expansion)

The term "culturing and expanding" means culturing that causes growth of the nephron progenitor cells while retaining the characteristics of the nephron progenitor cells. In other words, the cultured and expanded nephron progenitor cells can be differentiated in vitro into organ structures such as glomerulus-like structures or renal tubule-like structures of the kidney. The medium of the present aspect is capable of culturing and expanding nephron progenitor cells while retaining the characteristics of the nephron progenitor cells, even upon repeated subcultures.

### (Medium)

The medium of the present aspect of the invention may be a medium prepared by adding a GSK-3β inhibitor, a ROCK inhibitor, and at least one fibroblast growth factor selected from the group consisting of FGF9 and FGF20 to a basal medium used in animal cell culture. Further, the medium may also be a medium prepared by adding not only a GSK-3β inhibitor, a ROCK inhibitor, and at least one fibroblast growth factor selected from the group consisting of FGF9 and FGF20, but also a JAK inhibitor, to a basal medium used in animal cell culture.

### «Basal Medium»

There are no particular limitations on the basal medium, and any medium typically used in animal cell culture may be used without any particular restrictions. Examples of the basal medium include, but are not limited to, IMDM, Medium 199, Eagle's Minimum Essential Medium (EMEM), αMEM, Dulbecco's Modified Eagle Medium (DMEM), Ham's F12 (F12) medium, RPM11640 medium, Fischer's medium, and mixed media thereof. The medium may contain serum (such as fetal bovine serum (FBS)), or may be serum-free. If necessary, the medium may contain one or more serum replacements such as albumin, transferrin, KnockOut Serum Replacement (KSR), (a serum replacement for ES cell culture) (Invitrogen Corporation), N2 supplement (Invitrogen Corporation), B27 Supplement (Invitrogen Corporation), fatty acids, insulin, collagen precursors, trace elements, 2-mercaptoethanol and 3'-thiolglycerol. Further, the medium may also contain one or more substances such as lipids, amino acids, L-glutamine, GlutaMAX (Invitrogen Corporation), non-essential amino acids (NEAA), vitamins, growth factors, antibiotics, antioxidants, pyruvic acid, buffers, inorganic salts, or equivalents thereof.

The basal medium may be, for example, a medium prepared by adding amino acids, non-essential amino acids, and a serum replacement and the like to a mixed medium of DMEM/F12 (for example, a mixed medium prepared by mixing DMEM:F12 in a ratio of 1:1).

Supplements may also be added to the basal medium as appropriate. Examples of these supplements include AS401 (StemFit for Differentiation, Ajinomoto Healthy Supply Co., Inc.) and B27 (B-27 Supplement, minus vitamin A (Invitrogen Corporation). The basal medium preferably contains 10% by volume of AS401 relative to the total volume of the basal medium. In this description, a medium containing 10% by volume of AS401 relative to the total volume of the medium is sometimes referred to as a medium containing 10% added AS401.

The basal medium may be, for example, a medium containing 10% AS401 added to a mixed medium of DMEM/F12. For example, the basal medium may be a mixed medium of DMEM/F12 to which 10% AS401 and GlutaMAX (Invitrogen Corporation) have been added. DMEM/F12 Glutamax (Thermo Fischer Scientific Inc.) may be used as the mixed medium of DMEM/F12 containing added GlutaMAX. The basal medium may be DMEM/F12 Glutamax containing 10% added AS401. Using a basal medium containing 10% added AS401 improves the growth rate of the nephron progenitor cells, the shape of the cell mass, and the expression of NPC markers (SIX2 and OSR1) and the renal protection factor (VEGFA) and the like.

The medium of the present aspect of the invention contains a GSK-3β inhibitor, a ROCK inhibitor, and at least one fibroblast growth factor selected from the group consisting of FGF9 and FGF20.

### <<GSK-3β Inhibitor>>

The medium of the present aspect contains a GSK-3β inhibitor. The GSK-3β inhibitor is a substance that inhibits the function of GSK (Glycogen Synthase Kinase) 3β, for example, the kinase activity (such as the phosphorylation of β-catenin). Examples of the GSK-3β inhibitor include the indirubin derivative BIO (alternative name: GSK-3β inhibitor IX: 6-bromoindirubin-3'-oxime, the maleimide derivatives SB216763 (3-(2,4-dichlorophenyl)-4-(1-methyl-1H-indol-3-yl)-1H-pyrrole-2,5-dione) and SB415286 (3-[(3-chloro-4-hydroxyphenyl)amino]-4-(2-nitrophenyl)-1H-pyrrole-2,5-dione), the phenyl α-bromomethyl ketone compound GSK-3β inhibitor VII (4-dibromoacetophenone), the cell membrane-permeable phosphorylation peptide L803-mts (alternative name: GSK-3β Peptide Inhibitor; Myr-N-GKEAPPAPPQSpP-NH2), and CHIR99021 (6-[2-[4-(2,4-dichloropheny1)-5-(4-methy1-1H-imidazol-2-y1)pyrimidin-2-ylamino]ethylamino]-pyridine-3-carbonitrile), and derivatives of these substances. These compounds are available from Stemgent Inc., Calbiochem Corporation, and Biomol GmbH and the like, or may be prepared in-house. Other examples of the GSK-3β inhibitor include antisense nucleic acids, RNA-interference-inducing nucleic acids (such as siRNA) and dominant negative mutants against GSK-3β, and expression vectors or the like of one of these substances.

A single type of the GSK-3β inhibitor may be used alone, or a combination of two or more types may be used. One example of a preferred GSK-3β inhibitor is CHIR99021. The concentration of the GSK-3β inhibitor in the medium may be selected appropriately in accordance with the type of GSK-3β inhibitor used. For example, the GSK-3β inhibitor is preferably used at a concentration in the vicinity of IC50. In the case where the GSK-3β inhibitor is CHIR99021, the concentration of the GSK-3β inhibitor in the medium is, for example, typically within a range from 0.01 to 100 µM, and is preferably from 0.1 to 10 µM, more preferably from 0.5 to 3 µM, and particularly preferably from 0.5 to 1.5 µM.

### <<ROCK Inhibitor>>

The medium of the present aspect contains a ROCK inhibitor. The ROCK inhibitor is a substance that inhibits the function of Rho-kinase (ROCK). Examples of the ROCK inhibitor include Y-27632 (for example, see Ishizaki et al., Mol. Pharmacol. 57, 976 to 983 (2000), and Narumiya et al., Methods Enzymol. 325, 273 to 284 (2000), Fasudil/HA1077 (for example, see Uenata et al., Nature 389: 990 to 994 (1997)), H-1152 (for example, see Sasaki et al., Pharmacol. Ther. 93: 225 to 232 (2002)), Wf-536 (for example, see Nakajima et al., Cancer Chemother Pharmacol. 52(4): 319 to 324 (2003)), and derivatives of these substances; as well as antisense nucleic acids, RNA-interference-inducing nucleic acids (such as siRNA) and dominant negative mutants against ROCK, and expression vectors or the like of one of these substances. Other conventional low-molecular weight compounds may also be used as the ROCK inhibitor (for example, see U.S. Patent Publication Nos. 2005/0209261, 2005/0192304, 2004/0014755, 2004/0002508, 2004/0002507, 2003/0125344 and 2003/0087919, and International Patent Publication Nos. 2003/062227, 2003/059913, 2003/062225, 2022/076976 and 2004/039796.

A single type of the ROCK inhibitor may be used alone, or a combination of two or more types may be used. One example of a preferred ROCK inhibitor is Y-27632. The concentration of the ROCK inhibitor in the medium may be selected appropriately in accordance with the type of ROCK inhibitor used. For example, the ROCK inhibitor is preferably used at a concentration in the vicinity of IC50. In the case where the ROCK inhibitor is Y-27632, the concentration of the ROCK inhibitor in the medium is, for example, typically within a range from 0.1 to 100 µM, and is preferably from 1 to 75 µM, and more preferably from 5 to 50 µM.

The medium of the present aspect also contains at least one fibroblast growth factor selected from the group consisting of FGF9 and FGF20.

### «FGF9»

The medium of the present aspect may contain fibroblast growth factor (FGF) 9. There are no particular limitations on the organism from which the FGF9 is derived. For example, human FGF9 may be used as the FGF9. Examples of the human FGF9 (NCBI Gene ID: 2254) include proteins having the amino acid sequence of the NCBI accession number: NP_002001.1. The FGF9 may also be a fragment or functional variant of the FGF9, provided the activity that promotes the growth of the nephron progenitor cells is retained. A commercially available substance may be used as the FGF9, or a protein purified from cells or a protein produced by gene recombination may be used. Further, FGF20 belongs to the same sub-family as FGF9, and based on the finding that FGF20 exhibits an action that overlaps the action of FGF9 in nascent kidneys in mice (Barak, H. et al. FGF9 and FGF20 Maintain the Stemness of Nephron Progenitors in Mice and Man. Dev. Cell 22, 1191 to 1207 (2012)), it is thought that FGF20 can be used as a substitute for FGF9.

The concentration of FGF9 in the medium is, for example, typically within a range from 1 to 800 ng/ml, and is preferably from 5 to 500 ng/ml, more preferably from 10 to 400 ng/ml, and even more preferably from 100 to 300 ng/ml.

### <<FGF20>>

The medium of the present aspect may contain FGF20, either instead of the FGF9, or together with the FGF9. As described above, FGF20 and FGF9 are known to have similar activity in nascent kidneys, and can be mutually interchanged. There are no particular limitations on the organism from which the FGF20 is derived. For example, human FGF20 may be used as the FGF20. Examples of the human FGF20 (NCBI Gene ID: 26281) include proteins having the amino acid sequence of the NCBI accession number: NP_062825.1. The FGF20 may also be a fragment or functional variant of the FGF20, provided the activity that promotes the growth of the nephron progenitor cells is retained. A commercially available substance may be used as the FGF20, or a protein purified from cells or a protein produced by gene recombination may be used.

The concentration of FGF20 in the medium is, for example, typically within a range from 1 to 800 ng/ml, and is preferably from 5 to 500 ng/ml, more preferably from 10 to 400 ng/ml, and even more preferably from 100 to 300 ng/ml.

The medium of the present aspect may contain either one or both of FGF9 and FGF20. In those cases where the medium of the present aspect contains both FGF9 and FGF20, the concentration of the combination of FGF9 and FGF20 is, for example, typically within a range from 1 to 800 ng/ml, and is preferably from 5 to 500 ng/ml, more preferably from 10 to 400 ng/ml, and even more preferably from 100 to 300 ng/ml.

### <<Other Components>>

In addition to the components described above, the medium of the present aspect may also contain one or more other components. Examples of these other components include a JAK inhibitor.

### - JAK Inhibitor

The medium of the present aspect may contain a JAK inhibitor. A JAK inhibitor is a substance that inhibits the activity of one or more enzymes of the Janus kinase family (such as JAK1, JAK2, JAK3 and TYK2). By inhibiting the enzyme activity of the Janus kinase family, the JAK inhibitor inhibits signal transduction of the JAK-STAT system. By including a JAK inhibitor in the medium in addition to the GSK-3β inhibitor, the ROCK inhibitor, and the one or more fibroblast growth factors selected from the group consisting of FGF9 and FGF20, growth of the nephron progenitor cells can be better promoted. There are no particular limitations on the JAK inhibitor, provided it is capable of inhibiting the activity of one or more enzymes of the Janus kinase family. The JAK inhibitor preferably inhibits the enzyme activity of at least one enzyme selected from the group consisting of JAK1, JAK2 and JAK3, and an inhibitor that inhibits the enzyme activity of JAK2 (namely, a JAK2 inhibitor) is particularly preferred.

There are no particular limitations on the JAK3 inhibitor, provided it is capable of inhibiting the activity of JAK3. Examples of the JAK3 inhibitor include, but are not limited to, TCS21311 (CAS number: 1260181-14-3), WH1-P154 (CAS number: 211555-04-3), PF-06651600 (CAS number: 1792180-81-4), FM-381 (CAS number: 2226521-65-7), CP690550 (CAS number: 540737-29-9), 7H-pyrrolo[2,3-d]pyrimidine-5-carboxylic acid, 4-[3-[(2-methyl-1-oxo-2-propen-1-yl)amino]phenyl-, ethyl ester, JAK3 Inhibitor IV (Cas number: 58753-54-1), ZM449829 (CAS number: 4452-06-6), AZD1480 (CAS number: 935666-88-9), and Selective JAK3 Inhibitor 1 (CAS number: 1443235-95-7), as well as derivatives of these substances. Among these, TCS21311 is preferred as the JAK3 inhibitor.

There are no particular limitations on the JAK2 inhibitor, provided it is capable of inhibiting the activity of JAK2. Examples of the JAK2 inhibitor include, but are not limited to, NVP-BSK805 2HCl (CAS number: 1942919-79-0), TG101209 (CAS number: 936091-14-4), TG101348 (CAS number: 936091-26-8), 1,2,3,4,5,6-hexabromocyclohexane (CAS number: 1837-91-8), CEP33779 (CAS number: 1257704-57-6), and NSC33994 (CAS number: 82058-16-0), as well as derivatives of these substances. Among these, TG101348 is preferred as the JAK2 inhibitor.

The JAK inhibitor may be a JAK2/3 inhibitor. A JAK2/3 inhibitor is an inhibitor that inhibits JAK2 and JAK3. Examples of the JAK2/3 inhibitor include, but are not limited to AG490 (CAS number: 133550-30-8), AT9283 (CAS number: 896466-04-9) and LY3009104 (CAS number: 1187594-09-7), as well as derivatives of these substances.

The JAK inhibitor may be a JAK1/2 inhibitor. A JAK1/2 inhibitor is an inhibitor that inhibits JAK1 and JAK2. Examples of the JAK1/2 inhibitor include, but are not limited to CYT387 (CAS number: 1056634-68-4), S-Ruxolitinib (INCB018424) (CAS number: 941685-37-6) and LY2784544 (CAS number: 1229236-86-5), as well as derivatives of these substances.

The JAK inhibitor is not limited to the low-molecular weight compounds described above, and may also be an antisense nucleic acid, RNA-interference-inducing nucleic acids (such as siRNA) or dominant negative mutant against the Janus kinase family (JAK1, JAK2, JAK3 and TYK2), or an expression vector or the like of one of these substances.

A single type of the JAK inhibitor may be used alone, or a combination of two or more types may be used. The JAK inhibitor is preferably a JAK2 inhibitor, and is more preferably TG101348.

The concentration of the JAK inhibitor in the medium of the present aspect may be selected appropriately in accordance with the type of JAK inhibitor used. For example, the JAK inhibitor is preferably used at a concentration in the vicinity of IC50. For example, the JAK inhibitor may be used at a concentration of at least 0.01 nM, at least 0.05 nM, at least 0.1 nM, at least 0.5 nM, or 1 nM or higher. The upper limit for the concentration of the JAK inhibitor is, for example, not more than 100 nM, not more than 50 nM, nor more than 30 nM, not more than 20 nM, not more than 10 nM, or 5 nM or lower. In the case where the JAK inhibitor is TG101348, the concentration of the TG101348 in the medium is, for example, within a range from 0.5 to 50 nM, from 1 to 30 nM, from 1 to 20 nM, from 1 to 10 nM, or from 1 to 5 nM.

The medium of the present aspect of the invention may also contain components other than those described above, provided the effects of the present invention are not impaired. The medium of the present aspect preferably does not contain any signal transduction inhibitors, enzyme inhibitors, cytokines, or growth factors or the like other than those described above.

In one embodiment, the medium of the present aspect contains a GSK-3β inhibitor, a ROCK inhibitor, and at least one fibroblast growth factors selected from the group consisting of FGF9 and FGF20. In one embodiment, the medium of the present invention contains a GSK-3β inhibitor, a ROCK inhibitor, FGF9 and a JAK inhibitor. In one embodiment, the medium of the present aspect contains a GSK-3β inhibitor, a ROCK inhibitor, FGF9 and a JAK2 inhibitor. In one embodiment, the medium of the present aspect contains CHIR99021, Y-27632 and FGF9. In one embodiment, the medium of the present aspect contains CHIR99021, Y-27632, FGF9 and TG101348. The above FGF9 may be substituted with FGF20.

By including the GSK-3β inhibitor, the ROCK inhibitor, and at least one fibroblast growth factors selected from the group consisting of FGF9 and FGF20, the medium of the present aspect is able to produce favorable growth of the nephron progenitor cells while retaining the differentiation potency of the nephron progenitor cells. By also including a JAK inhibitor in addition to the above components, the medium of the present aspect yields even more favorable growth of the nephron progenitor cells.

By using the medium of the present aspect of the invention, nephron progenitor cells can be grown favorably with good retention of the differentiation potency of the nephron progenitor cells, even when repeated subculturing of the nephron progenitor cells is conducted. Accordingly, the medium of the present aspect can be said to be a medium for subculturing nephron progenitor cells. Further, the medium of the present aspect can also be said to be a maintenance medium for nephron progenitor cells.

### <Method for Culturing and Expanding Nephron Progenitor Cells>

A second aspect of the present invention is a method for culturing and expanding nephron progenitor cells that includes a step of culturing nephron progenitor cells in the medium of the first aspect described above.

Examples of the nephron progenitor cells cultured in the method of the present aspect include the same cells as those mentioned above in the section entitled "(Nephron Progenitor Cells: NPC)" within the above description of the <Medium for Culturing and Expanding Nephron Progenitor Cells>. The nephron progenitor cells are preferably human nephron progenitor cells. The nephron progenitor cells are preferably cells that have been induced from pluripotent stem cells, and are more preferably cells induced from iPS cells. In one embodiment, the nephron progenitor cells are nephron progenitor cells that have been induced from human iPS cells.

There are no particular limitations on the culturing method used in the method of the present aspect, provided the medium of the first aspect described above is used. The nephron progenitor cells may be cultured under the type of culturing conditions typically used for culturing animal cells. There are no particular limitations on the culturing temperature, provided the nephron progenitor cells are able to be grown, but the temperature is typically within a range from 25 to 40°C, and preferably from 30 to 40°C. One specific example of the culturing temperature is a temperature of about 37°C. The nephron progenitor cells can typically be cultured in a CO₂-containing air atmosphere. The CO₂ concentration is typically within a range from about 0.3 to 5%, and is preferably from about 2 to 5%. A specific example of the CO₂ concentration is about 5%.

The culture may be an adherent culture or a suspension culture, but a suspension culture is preferred.

There are no particular limitations on the culture period, and any arbitrary period may be used. In the method of the present aspect, by using the medium of the first aspect, long-term culturing can be conducted with good retention of the characteristics of the nephron progenitor cells. In the case of long-term culturing, appropriate subculturing is preferred. By conducting repeated subculturing using the medium of the first aspect described above, culturing of nephron progenitor cells can be continued with good retention of the characteristics of the nephron progenitor cells. The subculturing can be conducted by extracting a cell mass of the nephron progenitor cells from the culture solution, and inoculating a fresh medium with the cell mass. During subculturing, a cell dispersion containing enzymes such as a protease, collagenase and DNase may be used to disperse the cell mass prior to inoculation of the fresh medium. There are no particular limitations on the length of the subculture, and for example, a period of about 2 to 10 days, or about 3-7 days may be used. In one embodiment, the method of the present aspect includes subculturing of nephron progenitor cells.

The method for culturing and expanding nephron progenitor cells according to the present aspect may include the steps (A) and (B) described below:
(A) a step of culturing nephron progenitor cells in the medium of the first aspect for 30 to 60 hours; and
(B) a step of culturing the cells obtained in the above step (A) in a medium containing a GSK-3β inhibitor and at least one fibroblast growth factor selected from the group consisting of FGF9 and FGF20, but containing no ROCK inhibitor.

In step (A), the nephron progenitor cells are cultured for 30 to 60 hours in the medium of the first aspect. The culture time in the medium of the first aspect may be about two days. Examples of the culture time in the medium of the first aspect include times within a range from 35 to 55 hours, or from 40 to 50 hours. This culture time represents the time from inoculation of the nephron progenitor cells in the medium of the first aspect.

In step (B), the cells obtained in step (A) are cultured in a medium containing a GSK-3β inhibitor and at least one fibroblast growth factor selected from the group consisting of FGF9 and FGF20, but containing no ROCK inhibitor (hereinafter referred to as a "ROCK inhibitor-free medium"). Examples of the medium used in step (B) include the medium of the first aspect with the ROCK inhibitor removed. The culture of step (B) can be initiated by replacing the medium used in the culture of step (A) with a ROCK inhibitor-free medium. In step (B), medium replacement may be conducted at a frequency of about once every two days. The medium used for medium replacement may be a ROCK inhibitor-free medium. Medium replacement in step (B) may be conducted about 1 to 5 times, about 2 to 4 times, twice, or three times. Because the medium used in step (B) does not contain a ROCK inhibitor, culturing can be conducted more cheaply than cases where a medium containing a ROCK inhibitor is used.

The method of the present aspect may also include a step (C) described below following the above step (B):
(C) a step of subculturing the cells obtained in step (B) in the medium according to the first aspect.

The method of the present aspect may also include steps (D) and (E) described below following the above step (C):
(D) a step of culturing the cells subcultured in the step (C) in the medium of the first aspect for 30 to 60 hours, and
(E) a step of culturing the cells obtained in the step (D) in a medium containing a GSK-3β inhibitor and at least one fibroblast growth factor selected from the group consisting of FGF9 and FGF20, but containing no ROCK inhibitor.

In step (D), culturing may simply be conducted for 30 to 60 hours in the medium used for the subculture of step (C). The culture time in step (D) may be about two days. Examples of the culture time in step (D) include times within a range from 35 to 55 hours, or from 40 to 50 hours. This culture time represents the time from the subculture of the nephron progenitor cells.

In step (E), the cells obtained in step (D) are cultured in a ROCK inhibitor-free medium. In step (E), the same ROCK inhibitor-free medium as that used in step (B) may be used. The culturing in step (E) may be conducted in the same manner as that of step (B).

The nephron progenitor cells used in the method of the present aspect may be cells differentially induced from pluripotent stem cells (such as iPS cells). The nephron progenitor cells may be, for example, cells obtained using the differentiation induction method disclosed in International Patent Publication No. 2018/216743.

Further, the nephron progenitor cells used in the method of the present aspect may be, for example, cells obtained using a method including steps (i) to (vi) described below:
(i) a step of culturing pluripotent stem cells in a medium containing FGF2, BMP4, a GSK-3β inhibitor, and retinoic acid or a derivative thereof;
(ii) a step of culturing the cells obtained in the step (i) in a medium containing FGF2, a GSK-3β inhibitor and BMP7;
(iii) a step of culturing the cells obtained in the step (ii) in a medium containing a GSK-3β inhibitor, BMP7 and a TGFβ inhibitor, but containing no FGF2;
(iv) a step of culturing the cells obtained in the step (iii) in a medium containing FGF2, a GSK-3β inhibitor, activin and a ROCK inhibitor;
(v) a step of culturing the cells obtained in the step (iv) in a medium containing retinoic acid or a derivative thereof, but containing no FGF9; and
(vi) a step of culturing the cells obtained in the step (v) in a medium containing a GSK-3β inhibitor and at least one fibroblast growth factor selected from the group consisting of FGF9 and FGF20, thereby inducing nephron progenitor cells from intermediate mesodermal cells.

### (Step (i))

In this step, a late posterior epiblast is induced from the pluripotent stem cells. The late posterior epiblast is characterized as cells that are positive for at least one marker among CDX1, OCT4, NANOG and E-CADHERIN (CDH1), and preferably positive for all of these markers. The late posterior epiblast is preferably also negative for EOMES and BRACHYURY.

In step (i), the pluripotent stem cells are separated using any conventional method known in the field, and then preferably cultured by adherent culture. Examples of the method used for separating the pluripotent stem cells include mechanical separation, and separation using a separation solution having protease and collagenase activity (for example, Accutase (TM) and Accumax (TM) (Innovative Cell Technologies, Inc.), or a separation solution having only collagenase activity. The separation method is preferably a method in which the cells are dissociated using a separation solution having protease activity and collagenase activity, and then mechanically finely dispersed into single cells. The pluripotent stem cells used in step (i) are preferably colonies cultured to 70% to 80% confluence with respect to the dish being used.

The medium used in step (i) can be prepared by adding FGF2, BMP4, a GSK-3β inhibitor, and retinoic acid or a derivative thereof to a basal medium typically used in the culture of animal cells. The medium used in step (i) may also contain a ROCK inhibitor. In such cases, the medium used in step (i) can be prepared by adding the ROCK inhibitor as well as the components described above to the basal medium. The types of basal media described above may be used as the basal medium, and the basal medium may either contain serum, or be serum-free. If necessary, the basal medium may contain one or more serum replacements, lipids, amino acids, vitamins, growth factors, low-molecular weight compounds, antibiotics, antioxidants, pyruvic acid, buffers, and inorganic salts or the like.

The same types of ROCK inhibitor as those described above may be used as the ROCK inhibitor. An example of a preferred ROCK inhibitor is Y-27632. The concentration of the ROCK inhibitor used in step (i) may be selected appropriately by a person skilled in the art in accordance with the type of ROCK inhibitor used. In the case where the ROCK inhibitor is Y-27632, the concentration of the ROCK inhibitor is, for example, typically within a range from 0.1 to 100 µM, and is preferably from 1 to 75 µM, and more preferably from 5 to 50 µM.

The same types of GSK-3β inhibitor as those described above may be used as the GSK-3β inhibitor. An example of a preferred GSK-3β inhibitor is CHIR99021. The concentration of the GSK-3β inhibitor used in step (i) may be selected appropriately by a person skilled in the art in accordance with the type of GSK-3β inhibitor used. In the case where the GSK-3β inhibitor is CHIR99021, the concentration of the ROCK inhibitor is, for example, typically within a range from 0.01 to 100 µM, and is preferably from 0.1 to 10 µM, more preferably from 0.5 to 3 µM, and particularly preferably from 0.5 to 1.5 µM.

The FGF2 (basic FGF: bFGF) is preferably human FGF2. Examples of the human FGF2 include proteins having the amino acid sequence of the NCBI (National Center for Biotechnology Information) accession number: ABO43041.1. The FGF2 may also be a fragment or functional variant of FGF2, provided the differentiation induction activity is retained. A commercially available substance may be used as the FGF2, or a protein purified from cells or a protein produced by gene recombination may be used. The concentration of FGF2 used in this step is, for example, within a range from 1 to 1,000 ng/ml, preferably from 10 to 500 ng/ml, and more preferably from 50 to 250 ng/ml.

The BMP4 is preferably human BMP4. Examples of the human BMP4 include proteins having the amino acid sequence of the NCBI (National Center for Biotechnology Information) accession number: AAH20546.1. The BMP4 may also be a fragment or functional variant of BMP4, provided the differentiation induction activity is retained. A commercially available substance may be used as the BMP4, or a protein purified from cells or a protein produced by gene recombination may be used. The concentration of BMP4 used in this step is, for example, within a range from 0.1 to 100 ng/ml, preferably from 0.5 to 50 ng/ml, and more preferably from 0.5 to 5 ng/ml.

The retinoic acid may be retinoic acid itself, or may be a retinoic acid derivative that retains the differentiation induction potency of naturally occurring retinoic acid. Examples of these retinoic acid derivatives include 3-dehydroretinoic acid, 4-[[(5,6,7,8-tetrahydro-5,5,8,8-tetramethyl-2-naphthalenyl)carbonyl]amino]-benzoic acid (AM580) (Tamura K, et al., Cell Differ. Dev. 32: 17 to 26 (1990)), 4-[(1E)-2-(5,6,7,8-tetrahydro-5,5,8,8-tetramethyl-2-naphthalenyl)-1-propen-1-yl]-benzoic acid (TTNPB) (Strickland S, et al., Cancer Res. 43: 5268 to 5272 (1983)), as well as a compound disclosed in Tanenaga, K. et al., Cancer Res. 40: 914 to 919 (1980), retinol palmitate, retinol, retinal, 3-dehydroretinol, and 3-dehydroretinal.

The concentration of the retinoic acid or derivative thereof used in step (i) is, for example, within a range from 1 to 100 nM, preferably from 5 to 50 nM, and more preferably from 5 to 25 nM.

In step (i), there are no particular limitations on the culture temperature, but the temperature is typically about 30 to 40°C, and preferably about 37°C. The culture is conducted in a CO₂-containing air atmosphere. The CO₂ concentration is typically within a range from about 2 to 5%, and is preferably about 5%. The culture time of step (i) may be any time sufficient to achieve differential induction of a late posterior epiblast, but is typically one to two days, and preferably one day.

### (Step (ii))

In this step, a mesodermal-lineage primitive streak is induced from the late posterior epiblast. The mesodermal-lineage primitive streak is characterized as cells that are positive for CDX1 and BRACHYURY. The mesodermal-lineage primitive streak is preferably also negative for OCT4, NANOG, and CDH1.

In step (ii), a cell population obtained in the step (i) described above may be isolated and subjected to adherent culture in a separately prepared culture vessel that has been subjected to a coating treatment, or culture of the cells obtained by adherent culture in step (i) may simply be continued by replacing the medium.

The medium used in step (ii) can be prepared by adding FGF2, a GSK-3β inhibitor and BMP7 to a basal medium typically used in animal cell culture. The types of basal media described above may be used as the basal medium, and the basal medium may either contain serum, or be serum-free. If necessary, the basal medium may contain one or more serum replacements, lipids, amino acids, vitamins, growth factors, low-molecular weight compounds, antibiotics, antioxidants, pyruvic acid, buffers, and inorganic salts or the like.

The same type of FGF2 as that described above may be used. The concentration of the FGF2 used in step (ii) may be within the same range as the concentration described for step (i).

The same types of GSK-3β inhibitor as those described above may be used as the GSK-3β inhibitor. One example of a preferred GSK-3β inhibitor is CHIR99021. The concentration of the GSK-3β inhibitor used in step (ii) may be selected appropriately by a person skilled in the art in accordance with the type of GSK-3β inhibitor used, but is, for example, typically within a range from 0.01 to 100 µM, and is preferably from 0.1 to 10 µM, more preferably from 1 to 7.5 µM, and particularly preferably from 2 to 5 µM. The concentration of the GSK-3β inhibitor used in step (ii) is preferably higher than the concentration in step (i).

The BMP7 is preferably human BMP7. Examples of human BMP7 include proteins having the amino acid sequence of the NCBI (National Center for Biotechnology Information) accession number: NM_001719.2. The BMP7 may also be a fragment or functional variant of BMP7, provided the differentiation induction activity is retained. A commercially available substance may be used as the BMP7, or a protein purified from cells or a protein produced by gene recombination may be used.

The concentration of BMP7 used in step (ii) is, for example, within a range from 0.1 to 100 ng/ml, preferably from 0.5 to 50 ng/ml, and more preferably from 0.5 to 5 ng/ml.

In step (ii), there are no particular limitations on the culture temperature, but the temperature is typically about 30 to 40°C, and preferably about 37°C. The culture is conducted in a CO₂-containing air atmosphere. The CO₂ concentration is typically within a range from about 2 to 5%, and is preferably about 5%. The culture time of step (ii) may be any time sufficient to achieve differential induction of the mesodermal-lineage primitive streak, but is typically within a range from 10 hours to two days, one to two days, and preferably 0.5 to one day.

### (Step (iii))

In this step, a mesodermal-lineage late primitive streak is induced from the mesodermal-lineage primitive streak. The mesodermal-lineage late primitive streak is characterized as cells that are positive for CDX2 and BRACHYURY.

In step (iii), a cell population obtained in the step (ii) described above may be isolated and subjected to adherent culture in a separately prepared culture vessel that has been subjected to a coating treatment, or culture of the cells obtained by adherent culture in step (ii) may simply be continued by replacing the medium.

The medium used in step (iii) can be prepared by adding a GSK-3β inhibitor, BMP7 and a TGFβ inhibitor to a basal medium typically used in animal cell culture. The types of basal media described above may be used as the basal medium, and the basal medium may either contain serum, or be serum-free. If necessary, the basal medium may contain one or more serum replacements, lipids, amino acids, vitamins, growth factors, low-molecular weight compounds, antibiotics, antioxidants, pyruvic acid, buffers, and inorganic salts or the like.

The same types of GSK-3β inhibitor as those described above may be used as the GSK-3β inhibitor. The concentration of the GSK-3β inhibitor used in step (iii) may be within the same range as the concentration described for step (ii). The concentration of the GSK-3β inhibitor is, for example, typically within a range from 0.01 to 100 µM, and is preferably from 0.1 to 10 µM, more preferably from 1 to 7.5 µM, and particularly preferably from 2 to 5 µM.

The same type of BMP7 as that described above may be used. The concentration of the BMP7 used in step (iii) may be within the same range as the concentration described for step (ii).

The TGFβ inhibitor is a substance that inhibits signal transduction that proceeds from binding of TGFP to a receptor through to SMAD. Examples of the TGFβ inhibitor include substances that inhibit binding to the ALK family, which function as TGFβ receptors, and substances that inhibit phosphorylation of SMAD by the ALK family. Examples of such substances include Lefty-1 (NCBI Accession numbers include mouse: NM_010094, and human: NM_020997), SB431542 and SB202190 (R. K. Lindemann et al., Mol. Cancer, 2003, 2:20), SB505124 (GlaxoSmithKline plc), NPC30345, SD093, SD908 and SD208 (Scios Ltd.), LY2109761, LY364947 and LY580276 (Lilly Research Laboratories), A83-01 (WO 2009146408), and derivatives of these substances. The TGFβ inhibitor may be preferably A83-01.

There are no particular limitations on the concentration of the TGFP inhibitor used in step (iii), provided it enables inhibition of ALK. In those cases where the TGFβ inhibitor is A83-01, the concentration of the TGFβ inhibitor is, for example, typically within a range from 0.5 to 100 µM, preferably from 1 to 50 µM, and more preferably from 5 to 25 µM.

The medium used in step (iii) does not contain FGF2. Because the mesodermal-lineage late primitive streak is induced even without FGF2, culturing can be conducted more cheaply than methods in which a medium containing FGF2 is used.

In step (iii), there are no particular limitations on the culture temperature, but the temperature is typically about 30 to 40°C, and preferably about 37°C. The culture is conducted in a CO₂-containing air atmosphere. The CO₂ concentration is typically within a range from about 2 to 5%, and is preferably about 5%. The culture time of step (iii) may be any time sufficient to achieve differential induction of the mesodermal-lineage late primitive streak, but is typically one to three days, and preferably one to two days.

### (Step (iv))

In this step, a metanephric-lineage late primitive streak is induced from the mesodermal-lineage late primitive streak. The metanephric-lineage late primitive streak is characterized as cells that are positive for HOX11 and BRACHYURY.

In step (iv), a cell population obtained in the step (iii) described above may be isolated and subjected to adherent culture in a separately prepared culture vessel that has been subjected to a coating treatment, or culture of the cells obtained by adherent culture in step (iii) may simply be continued by replacing the medium.

The medium used in step (iv) can be prepared by adding a FGF2, a GSK-3β inhibitor, activin, and a ROCK inhibitor to a basal medium typically used in animal cell culture. The types of basal media described above may be used as the basal medium, and the basal medium may either contain serum, or be serum-free. If necessary, the basal medium may contain one or more serum replacements, lipids, amino acids, vitamins, growth factors, low-molecular weight compounds, antibiotics, antioxidants, pyruvic acid, buffers, and inorganic salts or the like.

The same types of FGF2 and GSK-3β inhibitor as those described above may be used. The concentrations of FGF2 and the GSK-3β inhibitor used in step (iv) may be within the same ranges as the concentrations described for step (ii). The concentration of the GSK-3β inhibitor used in step (iv) is, for example, typically within a range from 0.01 to 100 µM, and is preferably from 0.1 to 10 µM, more preferably from 1 to 7.5 µM, and particularly preferably from 2 to 5 µM. The concentration of FGF2 used in step (iv) is, for example, typically within a range from 1 to 1,000 ng/ml, preferably 30 ng/ml or higher, and more preferably from 30 to 100 ng/ml.

The activin may be human-derived activin, activin derived from another animal, or a functional variant of one of these activins. The activin may be activin A, activin B, or activin AB. Commercially available products from R&D Systems Inc. or the like may be used as the activin. An example of a preferred activin is activin A. The concentration of the activin used in step (iv) is, for example, typically within a range from 1 to 100 ng/ml, preferably from 5 to 50 ng/ml, and more preferably from 5 to 25 ng/ml.

The same types of ROCK inhibitors as those described above may be used as the ROCK inhibitor. An example of a preferred ROCK inhibitor is Y-27632. The concentration of the ROCK inhibitor used in step (iv) may be selected appropriately by a person skilled in the art in accordance with the type of ROCK inhibitor used. In those cases where the ROCK inhibitor is Y-27632, the concentration of the ROCK inhibitor is, for example, typically within a range from 0.1 to 100 µM, preferably from 1 to 75 µM, and more preferably from 5 to 50 µM.

The medium used in step (iv) may be a medium that does not contain BMP7. By using a medium not containing BMP7, step (iv) can be conducted more cheaply.

In step (iv), there are no particular limitations on the culture temperature, but the temperature is typically about 30 to 40°C, and preferably about 37°C. The culture is conducted in a CO₂-containing air atmosphere. The CO₂ concentration is typically within a range from about 2 to 5%, and is preferably about 5%. The culture time of step (iv) may be any time sufficient to achieve differential induction of the metanephric-lineage late primitive streak, but is typically one to five days, and preferably three days.

### (Step (v))

In this step, a late posterior intermediate mesoderm is induced from the metanephric-lineage late primitive streak. The intermediate mesoderm is characterized as cells that are positive for OSR1, HOX11, and WT1.

In step (v), a cell population obtained in the step (iv) described above may be isolated and subjected to adherent culture in a separately prepared culture vessel that has been subjected to a coating treatment, or culture of the cells obtained by adherent culture in step (iv) may simply be continued by replacing the medium.

The medium used in step (v) can be prepared by adding retinoic acid or a derivative thereof to a basal medium typically used in animal cell culture. The medium used in step (v) may also contain a BMP inhibitor. In such cases, the medium used in step (v) can be prepared by adding a BMP inhibitor to the basal medium as well as the above component. The types of basal media described above may be used as the basal medium, and the basal medium may either contain serum, or be serum-free. If necessary, the basal medium may contain one or more serum replacements, lipids, amino acids, vitamins, growth factors, low-molecular weight compounds, antibiotics, antioxidants, pyruvic acid, buffers, and inorganic salts or the like.

A BMP inhibitor is a substance that inhibits the function of BMP. Examples of the BMP inhibitor include protein-based inhibitors such as Chordin, Noggin and Follistatin, Dorsomorphin (namely, 6-[4-(2-piperidin-1-yl-ethoxy)phenyl]-3-pyridin-4-yl-pyrazolo[1,5-a]pyrimidine) and derivatives thereof (P. B. Yu et al. (2007), Circulation, 116:II_60; P. B. Yu et al. (2008), Nat. Chem. Biol., 4: 33 to 41; and J. Hao et al. (2008), PLoS ONE, 3(8): e2904), and LDN193189 (namely, 4-(6-(4-(piperazin-1-yl)phenyl)pyrazolo[1,5-a]pyrimidin-3-yl)quinoline).

An example of a preferred BMP inhibitor is Noggin. In those cases where the BMP inhibitor is Noggin, the concentration of the BMP inhibitor used in step (v) is, for example, typically within a range from 1 to 100 ng/ml, and preferably from 10 to 50 ng/ml.

The medium used in step (v) does not contain FGF9. Because the late posterior intermediate mesoderm is induced even without FGF9, culturing can be conducted more cheaply than methods in which a medium containing FGF9 is used. The medium used in step (v) need not contain FGF20.

In step (v), there are no particular limitations on the culture temperature, but the temperature is typically about 30 to 40°C, and preferably about 37°C. The culture is conducted in a CO₂-containing air atmosphere. The CO₂ concentration is typically within a range from about 2 to 5%, and is preferably about 5%. The culture time of step (v) may be any time sufficient to achieve differential induction of the late posterior intermediate mesoderm, but is typically one to three days, and preferably two days.

### (Step (vi))

In this step, nephron progenitor cells are induced from the late posterior intermediate mesoderm. The nephron progenitor cells are positive for OSR1, and also positive for HOX11, WT1, SIX2 and SALL1.

In step (vi), a cell population obtained in the step (v) described above may be isolated and subjected to adherent culture in a separately prepared culture vessel that has been subjected to a coating treatment, or culture of the cells obtained by adherent culture in step (v) may simply be continued by replacing the medium.

The medium used in step (vi) can be prepared by adding a GSK-3β inhibitor and at least one fibroblast growth factor selected from the group consisting of FGF9 and FGF20 to a basal medium typically used in animal cell culture. The medium used in step (vi) may also contain heparin. In such cases, the medium used in step (vi) can be prepared by adding the heparin as well as the components described above to the basal medium. The types of basal media described above may be used as the basal medium, and the basal medium may either contain serum, or be serum-free. If necessary, the basal medium may contain one or more serum replacements, lipids, amino acids, vitamins, growth factors, low-molecular weight compounds, antibiotics, antioxidants, pyruvic acid, buffers, and inorganic salts or the like.

The heparin is preferably in the form of a salt. Examples of heparin salts include salts with an alkali metal such as lithium, sodium or potassium; salts with an alkaline earth metal such as calcium, barium or magnesium; salts with other metals such as aluminum, zinc, copper or iron; ammonium salts; salts with organic bases; and salts with amino acids. Among the various possibilities, the heparin is preferably an alkali metal salt, and is more preferably a sodium salt.

The concentration of the heparin used in step (vi) is, for example, typically within a range from 0.01 to 100 µg/ml, preferably from 0.05 to 50 µg/ml, and more preferably from 0.1 to 10 µg/ml.

The same types of GSK-3β inhibitor, FGF9 and FGF20 as those described above may be used. The concentration of the GSK-3β inhibitor is, for example, typically within a range from 0.01 to 100 µM, and is preferably from 0.1 to 10 µM, more preferably from 0.5 to 3 µM, and particularly preferably from 0.5 to 1.5 µM. The concentration of the FGF9 or FGF20 (or the total concentration of FGF9 and FGF20) is, for example, typically within a range from 1 to 500 ng/ml, from 10 to 300 ng/ml, or from 50 to 200 ng/ml.

In step (vi), there are no particular limitations on the culture temperature, but the temperature is typically about 30 to 40°C, and preferably about 37°C. The culture is conducted in a CO₂-containing air atmosphere. The CO₂ concentration is typically within a range from about 2 to 5%, and is preferably about 5%. The culture time of step (vi) may be any time sufficient to achieve differential induction of the nephron progenitor cells, but is typically one to five days, and preferably two days.

The basal medium used in steps (i) to (vi) described above may use the same types of basal media described above in the section entitled "<Medium for Culturing and Expanding Nephron Progenitor Cells>". For example, a medium prepared by adding B27, AS401, and GlutaMAX-I (Invitrogen Corporation) or the like to a mixed medium of DMEM/F12 may be used as the basal medium. One specific example of the basal medium may be prepared by adding B27 and 10% AS401 or 20% AS401 to DMEM/F12 Glutamax. A medium prepared by adding 10% AS401 to DMEM/F12 Glutamax is preferred.

There are no particular limitations on the culture vessels used in the above steps (i) to (vi). Examples of the culture vessels include 24-well plates, 12-well plates, Petri dishes, culture flasks, bioreactors, and cell culture bags and the like. The use of a culture vessel that enables adherent culture is preferred. The area of the cell adhesion surface (for example, the bottom surface of the culture vessel) of the culture vessel is, for example, typically within a range from 1 to 1,000 cm². A culture vessel having a cell adhesion surface area of at least 400 cm² may be used. The area of the cell adhesion surface of the culture vessel is, for example, within a range from 400 to 800 cm², from 400 to 700 cm², or from 400 to 600 cm². In a differentiation induction method including steps (i) to (vi), nephron progenitor cells can be differentially induced even when the type of large-volume couture vessel described above is used. By using a large-volume culture vessel, the cell yield of the nephron progenitor cells can be increased. In steps (i) to (vi), the culture vessel may be changed at the time of medium replacement, or at the time of cell inoculation. For example, a medium-volume culture vessel (for example with a cell adhesion surface area of 50 to 200 cm²) may be used in steps (i) to (iii), with a large-volume culture vessel (for example, with a cell adhesion surface area of 400 cm² or greater) then being used in steps (iv) to (vi).

Adherent culture means culture conducted in a state where the cells are adhered to a culture substrate, and may mean, for example, culturing in a culture vessel that has been subjected to a coating treatment. The coating agent is preferably an extracellular matrix, and examples include substances such as collagen, proteoglycan, fibronectin, hyaluronic acid, tenascin, entactin, elastin, fibrillin and laminin, and fragments thereof. These extracellular matrices may be used in combination, and for example, may include products prepared from cells such as BD Matrigel (a registered trademark). The extracellular matrix is preferably laminin or a fragment thereof. In the present invention, laminin refers to a protein having a heterotrimer structure having one α-chain, one β-chain, and one γ-chain, and is an extracellular matrix protein in which isoforms exists with different compositions for the subunit chains. Laminin has about 15 types of isoforms in which the heterotrimer is composed of combinations of 5 types of α-chains, 4 types of β-chains, and 3 types of γ-chains. Examples include, but are not limited to, combinations in which the α-chain may be α1, α2, α3, α4 or α5, the β-chain may be β1, β2, β3 or β4, and the γ-chain may be γ1, γ2 or γ3. The laminin is more preferably laminin 511, which is composed of α5, β1 and γ1 (Nat Biotechnol 28, 611-615 (2010)). The laminin may also be a fragment, and there are no particular limitations on the fragment, provided it has integrin-binding activity. For example, the fragment may be an E8 fragment (laminin 511E8), which is a fragment obtained by digestion with elastase (EMBO J., 3:1463 to 1468, 1984, J. Cell Biol., 105: 589 to 598, 1987, and WO 2011/043405). Laminin 511E8 is commercially available, and can be procured, for example, from Nippi, Inc.

The nephron progenitor cells obtained using the steps (i) to (vi) described above can be cultured and expanded by culturing in the medium according to the first aspect. Alternatively, the nephron progenitor cells obtained using steps (i) to (vi) can be cultured and expanded by supplying the cells to a method including steps (A) and (B) described above, and optionally also including steps (C) to (E). In steps (A) to (E), similar culture vessels to those described above for use in steps (i) to (vi) may be used.

Because the method of the present aspect of the invention cultures nephron progenitor cells using the medium of the first aspect, the nephron progenitor cells can be grown favorably with good retention of their differentiation potency. Further, because the differentiation potency of the nephron progenitor cells is retained even upon subculturing, the nephron progenitor cells can be retained for long periods. Nephron progenitor cells cultured and expanded using the method of the present aspect may be administered to subjects having kidney disease, and used for treating the kidney disease. Further, the nephron progenitor cells can be used as a pharmaceutical composition for treating or preventing kidney disease. Furthermore, because nephron progenitor cells cultured using the method of the present aspect retain renal organoid formation capability, they can be used for producing the types of renal organoids described below.

### <Method for Producing Renal Organoids>

A third aspect of the present invention is a method for producing renal organoids. The method for producing renal organoids according to this aspect includes a step of culturing and expanding nephron progenitor cells using the method of the second aspect described above (a culturing and expansion step), and a step of differentiating the cultured and expanded nephron progenitor cells into renal organoids (a differentiation step).

### (Culturing and Expansion Step)

The culturing and expansion step is conducted using the method of the second aspect described above. This step enables the nephron progenitor cells to be grown favorably to achieve the desired number of cells, with good retention of the differentiation potency.

### (Differentiation Step)

The differentiation from the nephron progenitor cells into renal organoids may be achieved using a conventional method. For example, a method such as that reported in Nature, 526, 564 to 568 (2015) may be used. For example, a cell mass of the nephron progenitor cells obtained in the preceding culturing and expansion step may be co-cultured with feeder cells such as 3T3-Wnt4 cells, with fetal mouse spinal cord cells, or with fetal mouse kidney cells. Alternatively, a cell mass of the nephron progenitor cells may be subjected to semi-gas phase culture (and preferably gas-liquid culture: Nature, 526, 564 to 568 (2015)) using a basal medium containing a GSK-3β inhibitor. The medium used in the semi-gas phase culture may also contain FGF9 and FGF2 and the like, in addition to the GSK-3β inhibitor. Examples of the basal medium, the GSK-3β inhibitor and the FGF2 include the same substances as those mentioned above. An example of a preferred basal medium is KSR. An example of a preferred GSK-3β inhibitor is CHIR99021. An example of a preferred FGF2 is human FGF2. An example of a preferred FGF9 is human FGF9. Examples of human FGF9 include proteins having the amino acid sequence disclosed in NCBI accession number: NP_002001.1. The FGF9 may also be a fragment or functional variant of FGF9, provided the fragment or variant exhibits differentiation induction activity into renal organoids. A commercially available substance may be used as the FGF9, or a protein purified from cells or a protein produced by gene recombination may be used. In the above description, the FGF9 may be substituted with FGF20. Alternatively, a combination of FGF9 and FGF20 may be used.

The culture conditions in the differentiation step may employ the types of culture conditions typically used in animal cell culture. Although there are no particular limitations on the culture temperature, provided the differential induction into renal organoids can be achieved, the temperature is typically within a range from 25 to 40°, and preferably from 30 to 40°C. One specific example of the culture temperature is about 37°C. The CO₂ concentration may be typically set to a value within a range from about 0.3 to 5%, and preferably from about 2 to 5%. A specific example of the CO₂ concentration is about 5%.

Renal organoids obtained using the production method of the present aspect of the invention may be administered to subjects having kidney disease, and used for treating the kidney disease. Further, the renal organoids can be used as a pharmaceutical composition for treating or preventing kidney disease.

### (Other Steps)

The production method of the present aspect may also include one or more other steps in addition to the steps described above. An example of another step is a step, conducted prior to the above culturing and expansion step, of inducing nephron progenitor cells from pluripotent stem cells. This induction of nephron progenitor cells from pluripotent stem cells may be conducted using conventional methods.

### <Other Aspects>

The present invention also provides a pharmaceutical composition containing either the nephron progenitor cells obtained using the method of the second aspect or the renal organoids obtained using the production method of the third aspect. The present invention also provides a therapeutic agent for kidney disease containing the nephron progenitor cells or the renal organoids. The present invention also provides a method for treating or preventing kidney disease that includes a step of administering a therapeutically effective amount of the nephron progenitor cells or the renal organoids to either a subject having kidney disease or a subject at risk of kidney disease. The present invention also provides a method for producing cells for treating kidney disease that includes a step of culturing nephron progenitor cells in the medium according to the first aspect.

Examples of the method used for administering the nephron progenitor cells to a subject requiring treatment or prevention of kidney disease include: a method in which the nephron progenitor cells are formed into a sheet, and the sheet is attached to a kidney of the subject; a method in which a cell suspension prepared by suspending the nephron progenitor cells in physiological saline or the like is transplanted into a kidney of the subject; a method in which the nephron progenitor cells are subjected to three-dimensional culture (for example, Dev Cell. Sep 11, 2012; 23(3): 637 to 651), and the resulting cell mass is transplanted directly into a kidney of the subject; and a method in which the nephron progenitor cells are subjected to three-dimensional culture on a scaffold formed from Matrigel or the like, and the resulting cell mass is transplanted into a kidney of the subject. There are no particular limitations on the site of transplantation, provided the site is inside the kidney, but a site beneath the renal capsule is preferred. Examples of the method used for administering the above renal organoids to a subject requiring treatment or prevention of kidney disease include methods in which the renal organoids are transplanted in vivo (for example, intraperitoneally).

There are no particular limitations on the kidney disease that represents the treatment or prevention subject, and examples include acute kidney injury, chronic renal failure, and chronic kidney diseases that have not progressed to chronic renal failure. There are no particular limitations on the number of nephron progenitor cells or the size of the renal organoids that are transplanted, provided they survive the transplant, and these factors may be adjusted appropriately in accordance with factors such as the size of disease site, and the physique, age, and gender and the like of the administration subject.

### [Examples]

The present invention is described below in further detail using a series of examples, but the present invention is not limited to the following examples.

### [Materials and Methods]

### <Animal Experiments>

All animal experiments were conducted with the approval of the Kyoto University Animal Experiment Committee. NOD.CB17-Prkdc^{scid}/J mice were procured from Charles River Laboratories Japan, Inc. The mice were raised under specific pathogen free (SPF) conditions in an experimental animal facility of the iPS cell research center of Kyoto University. Food and water was freely available to the mice.

### induction of Nephron Progenitor Cells (hNPC) from Human iPS Cells>

The 4A6 line (derived from 201B7), which is a human iPS cell line derived from normal human subjects, was differentially induced into human iPS cell-derived NPC in a 24-well plate using the method disclosed in International Patent Publication No. 2018/216743, 100 µL of Accumax was then added, and the cells were then left to stand for 30 minutes in an incubator at 37°C with a CO₂ concentration of 5%. After the 30 minutes, the cells were suspended in 900 µL of 10% FBS. The cell count of the suspended cells was measured with a TC20, a quantity of cells required for flow cytometry was transferred to a 1.5 ml tube, and the tube was centrifuged at 200 g for 5 minutes. Following centrifugation, the supernatant was removed, and the cells were suspended in a ratio of 100 µL of 2% FBS per 1.0×10⁶ cells.

### <Purification of c-MET-Positive Cells>

Ten µL of human-HGFR/c-MET antibody to which the fluorescent substance APC had been bound (FAB 3582A, R&D Systems Inc.) was added to 100 µL of the 2% FBS suspension of the induced hNPC cells prepared above, and the resultant was left to stand for 30 minutes on ice. After 30 minutes, the sample was centrifuged at 200 g for 5 minutes, the supernatant was removed, the residue was washed with 1 mL of PBS, and the sample was once again centrifuged at 200 g for 5 minutes and the supernatant removed. This operation was conducted twice. The cells obtained following removal of the supernatant were suspended in DAPI that had been diluted 1,000-fold with 2% FBS, and following filtration through a 40 µm filter, flow cytometry was used to extract the c-MET-positive cells.

In this description, hNPC that have not undergone purification of the c-MET-positive cells are referred to as "hNPC (Bulk)". Further, hNPC that have undergone purification of the c-MET-positive cells are sometimes referred to as "hNPC (c-MET(+))".

### <Medium for Culturing and Expansion>

An hNPSR medium, a CFY medium, and a medium prepared by adding either DMSO or DMSO containing a dissolved JAK inhibitor (JAK2 inhibitor: TG101348 (final concentration: 3 nM)) to the CFY medium were used as test media for culturing and expansion.

The composition of the hNPSR medium is shown in Table 1. The hNPSR medium was prepared by adding the reagents shown in Table 1 to the basal medium shown in Table 1. The composition of the CFY medium is shown in Table 2. The CFY medium was prepared by adding the reagents shown in Table 2 to the basal medium shown in Table 2.

**[Table 1]**

| hNPSR Medium | | | |
|---|---|---|---|
| Basal medium: DMEMIF12 (1:1) (1×). Invitrogen, Cat. No. 11330-32 | | | |
| Reagent Name | Manufacturer | Cat. No. | Final concentration |
| GlutaMAX-I (100×) | Invitrogen | 35050-79 | 1× |
| MEM NEAA (100×) | Invitrogen | 11140-050 | 1× |
| 2-mercaptoethanol (55 mM) | Invitrogen | 21985-023 | 0.1 µM |
| Pen Strep (100×) | Invitrogen | 15140-122 | 1× |
| B-27 Supplement (50×), minus vitamin A | Invitrogen | 12587-010 | 1× |
| ITS Liquid Media Supplement (100×) | Sigma | 13146-5ML | 1× |
| BMP-7 | R&D | 354-BP-010 | 50 ng/mL |
| FGF-2 | Peprotech | 100-18B | 300 ng/mL |
| Heparin | Sigma | H3149-100KU | 1 µg/mL |
| Y-27632 | Enzo | ALX-270-333-M025 | 10 µM |
| Human LIF | Millipore | LIF1050 | 10 ng/mL |
| CHIR99021 | Reagents Direct | 27-H76 | 1 µM |
| LDN193189 | Reagents Direct | 36-F52 | 10 nM |
| A83-01 | STEM GENT | 04-0014 | 50 nM |

**[Table 2]**

| CFY Medium | | | |
|---|---|---|---|
| Basal medium: DMEM/F12 Glutamax medium, Thermo Fisher Scientific, Cat. No. 10565042 | | | |
| Reagent Name | Manufacturer | Cat. No. | Final concentration |
| Pen Strep (100×) | Invitrogen | 15140-122 | 1× |
| B-27 Supplement (50×), minus vitamin A | Invitrogen | 12587-010 | 1× |
| CHIR99021 | Reagents Direct | 27-H76 | 1 µM |
| Y-27632 | Enzo | ALX-270-333-M025 | 10 µM |
| FGF-9 | Peprotech | 100-23 | 200 ng/mL |

### <Cell Growth>

The hNPC were suspended in 50 µL of a test medium in sufficient amount to achieve 2.0×10⁴ cells per well, and the suspension was then used to inoculate a low-attachment 96-well plate (Nunc), and following centrifuging at 300 g for 3 minutes, the cells were subjected to a static culture in an incubator at 37°C and 5% CO₂. Six hours after starting the static culture, a cell mass had formed.

Subsequently, 48 hours after the start of the static culture, an additional 100 µL of the same medium as that used at the time of cell inoculation was administered to each well. Then, 48 hours after this additional administration of medium, 100 µL of supernatant was removed from each well, and a further 100 µL of fresh medium was added. Thereafter, the same operations were repeated every 48 hours. Seven days after starting this suspension culture, the cell mass in each well was transferred to a 1.5 ml tube, the supernatant was removed completely, 30 µL of Accumax (Innovative Cell Technologies, Inc.) was added, and the resultant was left to stand for 10 minutes in an incubator at 37°C and 5% CO₂. After 10 minutes, the sample was suspended in 470 µL of 10% FBS, and the cell count was measured with a TC20 (Bio-Rad Laboratories, Inc.).

### <Subculture>

A cell mass of the human iPS cell-derived NPC (hNPC) cultured in the test medium was transferred to a 1.5 ml tube, the supernatant was removed completely, 30 µL of Accumax (Innovative Cell Technologies, Inc.) was added, and the resultant was left to stand for 10 minutes in an incubator at 37°C and 5% CO₂. After 10 minutes, the sample was suspended in 470 µL of 10% FBS, and the cell count was measured with a TC20 (Bio-Rad Laboratories, Inc.). The human iPS cell-derived NPC were suspended in the test medium and then used to inoculate a low-attachment 96-well plate (Nunc) in an amount of 2.0×10⁴ cells per well. This low-attachment 96-well plate that had been inoculated with the cells was centrifuged at 300 g for 3 minutes, and the cells were then subjected to static culture in an incubator at 37°C and 5% CO₂. Then, 48 hours after starting this static culture, an additional 100 µL of the test medium was administered to each well. Subsequently, 48 hours after this additional administration, the above operations were repeated, with the cell count being measured and a subculture conducted.

### <Production of Renal Organoids>

A cell mass of hNPC that had been subcultured in the test medium were placed in a Transwell (Corning), and the surrounding medium was removed. Next, the Transwell with the cell mass thereon was set in a culture dish that had been filled with 5% KSR (Kock-out Serum Replacement) (Thermo Fisher Scientific) to which 200 ng/mL of FGF2 and 5 µM of CHIR99021 had been added in advance. After 48 hours, the medium was replaced with 5% KSR containing no added growth factors or low-molecular weight compounds. Thereafter, the medium was replaced every 48 hours with fresh 5% KSR containing no added growth factors or low-molecular weight compounds, and on the 10th day after starting the differentiation culture, the cells were fixed for 3 hours at 4°C using 4% PFA without removal from the Transwell. Following fixing, the medium was replaced with PBS, and after standing overnight at 4°C, immunostaining was conducted.

### <Confirmation of Expression of OSR1 and SIX2>

Human iPS cells were established which had an OSR1-GFP reporter gene in which a GFP coding region had been introduced at the OSR1 locus, and also had an introduced SlX2-tdTomato reporter gene in which the tdTomato coding region was linked to the SIX2 gene. Expression of OSR1 and SIX2 was confirmed by conducting a culture test using these human iPS cells, and detecting the respective fluorescence from the GFP and tdTomato using a fluorescence microscope.

### <Imaging of Renal Organoids>

Bright field images of renal organoids were captured using either a KEYENCE BZ-X700 or a confocal microscope (Zeiss LSM710). Image analysis was performed using Image J version 1.51j8 (National Institutes of Health) (C.A. Schneider, et al., Nat Methods. 9 (2012) 671 to 675.).

### <hNPC Administration Experiments>

Cisplatin that had been adjusted to a concentration of 1 mg/mL with physiological saline was injected subcutaneously into 8 to 12-week old male NOD.CB17-Prkdc^{scid}/J mice (hereinafter referred to as "NOD-SCID mice") at a dose of 21 mg/kg to create a cisplatin-induced acute kidney injury (AKI) model. Twenty four hours after the cisplatin administration, the left kidney was exposed extracorporeally under general anesthesia with isoflurane by making an approximately 1 cm skin incision in the lower left dorsal region of each NOD-SCID mouse, followed by an approximately 1 cm incision in the retroperitoneum. A 24-gauge Surflo indwelling needle was used to puncture the exposed kidney, and an outer tube was secured beneath the renal capsule. The renal capsule and the renal parenchyma were detached by injecting air or physiological saline with a syringe through the outer tube. Subsequently, the outer tube of the 24-gauge Surflo indwelling needle was removed, an hNPC (Bulk) cell mass (equivalent to about 3.0×10⁶ cells) that had been cultured and expanded for one or two passages in the CFY medium) was inserted from the same puncture site into the outer tube of an aspirated 22-gauge Surflo indwelling needle, and the cells were injected slowly into the kidney (cell transplantation). In the saline group, 15 µL of physiological saline was injected beneath the renal capsule instead of the hNPC (Bulk).

Following injection of the cells, the outer tube of the 22-gauge Surflo indwelling needle was removed, and following confirmation that there was no cell outflow or bleeding from the puncture site, the exposed kidney was returned to the retroperitoneum, and the retroperitoneum and skin were sutured with 4-0 blade silk sutures.

Ninety six hours after the cisplatin administration (72 hours after the cell transplantation), 100 to 200 µL of blood was collected from the subocular venous plexus and centrifuged to obtain the serum. Subsequently, using the serum obtained from the centrifugal separation, the serum creatinine (S-Cre) and blood urea nitrogen (BUN) were measured using a Fuji Drychem NX500.

### (Example 1)

Subcultures of hNPC (Bulk) were conducted using the CFY medium and the hNPSR medium, and the cell growth, expression of NPC markers, and renal organoid formation capability of the cultured cells were ascertained.

### <Maintenance Culture of hNPC (Bulk) in CFY Medium>

Subcultures of hNPC (Bulk) were conducted using the hNPSR medium or the CFY medium, and verification was made as to whether the expression of the NPC markers SIX2 and OSR1 was maintained in each subculture.

The results are shown in FIG. 1. In the case of the hNPC (Bulk) subcultured in the hNPSR medium, the expression of OSR1 was detected up until passage 3, but the expression of SIX2 was hardly detected from the passage 2 onward. In contrast, in the case of the hNPC (Bulk) subcultured in the CFY medium, expression of both OSR1 and SIX2 was detected in all of passages 1 to 3. These results confirmed that expression of the NPC markers was more readily maintained in the subculture using the CFY medium. In other words, it was confirmed that by using the CFY medium, hNPC (Bulk) could be subcultured while retaining their characteristics as NPC.

### <Production of Renal Organoids from hNPC (Bulk) Subcultured in CFY Medium>

Attempts were made to produce renal organoids from hNPC (Bulk) that had been subcultured using either the hNPSR medium or the CFY medium.

The results are shown in FIG. 2. When subculturing was conducted in the hNPSR medium, renal organoids were formed from the hNPC (Bulk) of passage 1. However, renal organoids were not formed from the hNPC (Bulk) of passage 2 or passage 3. In contrast, when subculturing was conducted in the CFY medium, renal organoids were formed from the hNPC (Bulk) of all of passages 1 to 3. These results confirmed that by using the CFY medium, hNPC (Bulk) could be subcultured while maintaining their ability to form renal organoids.

### <Cell Growth in Subculture of hNPC (Bulk) using CFY Medium>

Subcultures of hNPC (Bulk) were conducted using media obtained by adding DMSO to either the hNPSR medium or the CFY medium, and the cell counts were measured.

The results are shown in FIG. 3 and FIG. 4. FIG. 3 shows the cumulative cell count calculated from the cells in each subculture. The cumulative cell count in passage 1, when the cell count in passage 0 is deemed a, the volume of the culture solution of passage 0 is deemed b, the volume of the culture solution of P0 used for subculturing is deemed c, and the cell count of the cells grown in P1 is deemed d, can be calculated using the formula: [cumulative cell count in passage 1] = a×[d/(a×c/b)]×b/c. The cumulative cell count in the subsequent subcultures is calculated in a similar manner.

As illustrated in FIG. 3 and FIG. 4, the growth of hNPC (Bulk) was promoted regardless of whether the hNPSR medium or the CFY medium was used. The growth promotion effect on hNPC (Bulk) was higher in the hNPSR medium than in the CFY medium.

### (Example 2)

A subculture of hNPC (Bulk) was conducted using a medium prepared by adding a JAK inhibitor to the CFY medium, and the cell growth, expression of NPC markers, and renal organoid formation capability of the cultured cells were ascertained.

### <Cell Growth Promotion Effect provided by JAK Inhibitor>

Cultures of hNPC (Bulk) were conducted using either a medium prepared by adding DMSO to the CFY medium, or a medium prepared by adding 3 nM of TG101348 (a JAK2 inhibitor) to the CFY medium, and the cell counts were measured.

The results are shown in FIG. 5. In the case of the culture in the medium prepared by adding TG101348 to the CFY medium (CFY+TG(3)), cell growth was promoted more than in the medium prepared by adding DMSO to the CFY medium (CFY+DMSO). These results confirmed that addition of a JAK inhibitor improves the growth promotion effect on hNPC (Bulk) in the CFY medium.

### <Maintenance Culture of hNPC (Bulk) in CFY Medium with added JAK Inhibitor>

Subcultures of hNPC (Bulk) were conducted using either a medium prepared by adding DMSO to the CFY medium, or a medium prepared by adding 3 nM TG101348 (a JAK2 inhibitor) to the CFY medium, and verification was made as to whether the expression of the NPC markers OSR1 and SIX2 was maintained in each subculture.

The results are shown in FIG. 6. Expression of both OSR1 and SIX2 was maintained up until passage 3 in both the medium prepared by adding DMSO to the CFY medium (+DMSO) and the medium prepared by adding 3 nM TG101348 to the CFY medium (+TG(3)). These results confirmed that by using a medium prepared by adding a JAK inhibitor to the CFY medium, hNPC (Bulk) could be subcultured while retaining the characteristics of the NPC.

### <Production of Renal Organoids from hNPC (Bulk) Subcultured in CFY Medium with added JAK Inhibitor>

Subcultures of hNPC (Bulk) were conducted using either a medium prepared by adding DMSO to the CFY medium, or a medium prepared by adding 3 nM TG101348 (a JAK2 inhibitor) to the CFY medium, and attempts were made to produce renal organoids from the cells of passage 2.

The results are shown in FIG. 7. Renal organoids were formed from the cells of passage 2 in both the medium prepared by adding DMSO to the CFY medium (+DMSO) and the medium prepared by adding 3 nM TG101348 to the CFY medium (+TG(3)). These results confirmed that by using a medium prepared by adding a JAK inhibitor to the CFY medium, hNPC (Bulk) could be subcultured while maintaining their ability to form renal organoids.

### (Example 3)

A subculture of hNPC (c-MET(+)) was conducted using a medium prepared by adding a JAK inhibitor to the CFY medium, and the cell growth, expression of NPC markers, and renal organoid formation capability of the cultured cells were ascertained.

### <Cell Growth Promotion Effect of JAK Inhibitor in Subculture of hNPC (c-MET(+)) >

Subcultures of hNPC (c-MET(+)) were conducted using either a medium prepared by adding DMSO to the CFY medium, or a medium prepared by adding 3 nM TG101348 (a JAK2 inhibitor) to the CFY medium, cell counts were measured, and cumulative cell counts were calculated.

The results are shown in FIG. 8. In the case of culture in the medium prepared by adding TG101348 to the CFY medium (CFY+TG(3)), cell growth was better promoted in all passages compared with culture in the medium prepared by adding DMSO to the CFY medium (CFY+DMSO). These results confirmed that the addition of the JAK inhibitor improved the growth promotion effect of hNPC (c-MET(+)) in the CFY medium.

### <Maintenance Culture of hNPC (c-MET(+)) in CFY medium with added JAK inhibitor>

Subcultures of hNPC (c-MET(+)) were conducted using either a medium prepared by adding DMSO to the CFY medium, or a medium prepared by adding 3 nM TG101348 (a JAK2 inhibitor) to the CFY medium, and verification was made as to whether the expression of the NPC markers OSR1 and SIX2 was maintained in each subculture.

The results are shown in FIG. 9. Expression of both OSR1 and SIX2 was maintained up until passage 3 in both the medium prepared by adding DMSO to the CFY medium (+DMSO) and the medium prepared by adding 3 nM TG101348 to the CFY medium (+TG(3)). These results confirmed that by using a medium prepared by adding a JAK inhibitor to the CFY medium, hNPC (c-MET(+)) could be subcultured while retaining the characteristics of the NPC.

### <Production of Renal Organoids from hNPC (c-MET(+)) Subcultured in CFY Medium with added JAK Inhibitor>

Subcultures of hNPC (c-MET(+)) were conducted using either a medium prepared by adding DMSO to the CFY medium, or a medium prepared by adding 3 nM TG101348 (a JAK2 inhibitor) to the CFY medium, and attempts were made to produce renal organoids from the cells of passage 1.

The results are shown in FIG. 10. Renal organoids were formed from the cells of passage 1 in both the medium prepared by adding DMSO to the CFY medium (+DMSO) and the medium prepared by adding 3 nM TG101348 to the CFY medium (+TG(3)). These results confirmed that by using a medium prepared by adding a JAK inhibitor to the CFY medium, hNPC (c-MET(+)) could be subcultured while maintaining their ability to form renal organoids.

### (Example 4)

Cell masses of hNPC (Bulk) that had been cultured and expanded in the CFY medium were transplanted into the renal subcapsule of AKI mice, and the therapeutic effect of hNPC (Bulk) was ascertained.

The transplantation results of the NPC masses from passage 1 and passage 2 are shown in FIG. 11 and FIG. 12 respectively. In FIG. 11 and FIG. 12, "Normal" indicates a normal mouse, "Saline" indicates an AKI mouse to which physiological saline had been administered into the renal subcapsule, and "NPC transplant" indicates an AKI mouse in which a cell mass of hNPC (Bulk) had been transplanted into the renal subcapsule. The AKI mice to which physiological saline had been administered had significantly higher BUN and S-Cre numerical values than the normal mice, confirming renal dysfunction. On the other hand, in the mice transplanted with the hNPC (Bulk) cell mass, the BUN and S-Cre values were significantly lower for both the passage 1 hNPC and the passage 2 hNPC than the mice to which the saline solution had been administered, indicating an improvement in renal function. These results confirmed that by transplanting hNPC that had been cultured and expanded in the CFY medium, renal dysfunction could be improved.

In Examples 1 to 4 described above, NPC differentially induced by the differentiation induction method A described below were used, based on the method disclosed in International Patent Publication No. 2018/216743.

### <Differentiation Induction Method A>

1. Undifferentiated iPS cells were converted to single cells by an accutase treatment, the cells were then suspended in 500 µL of an AK02N medium (Ajinomoto) to which 10 µM of Y-27632 and 1.25 µL of iMatrix (Nippi) had been added, followed by plating on a 24-well plate at a density within a range from 1.0×10⁴ cells/well to 5.0×10⁴ cells/well, and the cells were then incubated for 24 hours at 37° C.
2. After 24 hours (day 1), medium replacement was conducted using a medium prepared using DMEM/F12 Glutamax (Thermo Fisher Scientific Inc.) supplemented with B27 (B-27 Supplement, minus vitamin A, Invitrogen) as the basal medium and then adding 1 µM of CHIR99021, 10 nM of retinoic acid, 1 ng/ml of BMP4 and 100 ng/ml of FGF2 to this basal medium (production of a late posterior epiblast ...1).
3. On day 2, medium replacement was conducted using a medium prepared by adding 3 µM of CHIR99021, 1 ng/ml of BMP7 and 100 ng/ml of FGF2 to the same basal medium as above (production of a mesodermal-lineage primitive streak ...2).
4. On day 3, medium replacement was conducted using a medium prepared by adding 3 µM of CHIR99021, 1 ng/ml of BMP7, 100 ng/ml of FGF2, and 10 µM of A83-01 to the same basal medium (production of a mesodermal-lineage late primitive streak ...3).
5. On day 4, day 5 and day 6, medium replacement was conducted using a medium prepared by adding 3 µM of CHIR99021, 1 ng/ml of BMP7, 100 ng/ml of FGF2, 10 ng/ml of activin and 30 µM of Y-27632 to the same basal medium (production of a metanephric-lineage late primitive streak ...4).
6. On day 7 and day 8, medium replacement was conducted using a medium prepared by adding 200 ng/ml of FGF9, 100 nM of retinoic acid and 25 ng/ml of Noggin to the same basal medium (production of a late posterior intermediate mesoderm ...5).
7. On day 9, day 10 and day 11, medium replacement was conducted using a medium prepared by adding 200 ng/ml FGF9 and 1 µM of CHIR99021 to the same basal medium (production of renal progenitor cells (nephron progenitor cells) ...6).

### (Example 5)

Using the protocol described below, an investigation was conducted as to whether hNPC was differentially induced from human iPS cells (hiPSC). The hiPSC used were OSR1-GFP/SIX2-tdTomato reporter human iPS cells derived from 201B7.

### <Method for Differentially Inducing hNPC from hiPSC (Differentiation Induction Method B)>

1. Undifferentiated hiPSC were converted to single cells using a TrypLE Select Enzyme (Gibco), and then using a medium prepared using DMEM/F12 Glutamax (Invitrogen) supplemented with B27 as the basal medium and adding 1 µM of CHIR99021, 10 nM of retinoic acid, 1 ng/ml of BMP4, 100 ng/ml of FGF2, 10 µM of Y-27632 and iMatrix-511 (Nippi) to this basal medium, plating was conducted at a density within a range from 1.0×10⁴ cells/well to 5.0×10⁴ cells/well, and the cells were then incubated for 24 hours at 37° C (production of a late posterior epiblast: step 1).
2. On day 1, medium replacement was conducted using a medium prepared by adding 5 µM of CHIR99021, 1 ng/ml of BMP7 and 100 ng/ml of FGF2 to the same basal medium as above (production of a mesodermal-lineage primitive streak: step 2).
3. On day 2, medium replacement was conducted using a medium prepared by adding 5 µM of CHIR99021, 1 ng/ml of BMP7, 100 ng/ml of FGF2 and 10 µM of A83-01 to the same basal medium as above (production of a mesodermal-lineage late primitive streak: step 3).
4. On day 3, medium replacement was conducted using a medium prepared by adding 5 µM of CHIR99021, 100 ng/ml of FGF2, 1 ng/ml of BMP7, 10 ng/ml of activin A and 30 µM of Y-27632 to the same basal medium (production of a metanephric-lineage late primitive streak: step 4).
5. On day 6, medium replacement was conducted using a medium prepared by adding 100 nM of retinoic acid, 200 ng/ml of FGF9 and 25 ng/ml of Noggin to the same basal medium (production of a late posterior intermediate mesoderm: step 5).
6. On day 8, medium replacement was conducted using a medium prepared by adding 1 µM of CHIR99021 and 200 ng/ml FGF9 to the same basal medium (production of hNPC: step 6).

Further, with the exception of replacing the 100 ng/ml of FGF2 in step 4 of the above differentiation induction method B (production of a metanephric-lineage late primitive streak: step 4) with any one of 30 ng/ml of FGF2; 30 ng/ml of FGF2 and 0.18 U/ml (1 µg/ml) of heparin; 10 ng/ml of FGF2; 10 ng/ml of FGF2 and 0.18 U/ml (1 µg/ml) of heparin; or 0 ng/ml of FGF2, hNPC were differentially induced from hiPSC using the same procedure as the differentiation induction method B.

For each of these differentially induced hNPC, the results of confirming the differentiation induction efficiency of SIX2-positive cells are illustrated in FIG.13. The percentages of SIX2-positive cells among the live cells not stained by DAPI are shown. When the FGF2 concentration in step 4 (production of a metanephric-lineage late primitive streak: step 4) was reduced to 10 ng/mL or less, the induction efficiency into hNPC decreased. The presence of heparin had no effect on the hNPC induction efficiency.

### (Example 6)

The hNPC were differentially induced from hiPSC in accordance with the protocol of the above differentiation induction method B.

Further, with the exceptions of replacing the 100 ng/ml of FGF2 in step 3 of the differentiation induction method B (production of a mesodermal-lineage late primitive streak: step 3) with either 30 ng/ml of FGF2 or 10 ng/ml of FGF2, and also replacing the 100 ng/ml of FGF2 in step 4 of the differentiation induction method B (production of a metanephric-lineage late primitive streak: step 4) with 30 ng/ml of FGF2, hNPC were differentially induced from hiPSC using the same procedure as the differentiation induction method B.

For each of these differentially induced hNPC, the results of confirming the differentiation induction efficiency of SIX2-positive cells are illustrated in FIG.14. The percentages of SIX2-positive cells among the live cells not stained by DAPI are shown. The concentration of FGF2 in step 3 (production of a mesodermal-lineage late primitive streak: step 3) had no effect on the hNPC induction efficiency.

### (Example 7)

The hNPC were differentially induced from hiPSC in accordance with the protocol of the above differentiation induction method B.

Further, with the exceptions of replacing the 200 ng/ml of FGF9 in step 5 of the differentiation induction method B (production of a late posterior intermediate mesoderm: step 5) with any one of 100 ng/ml of FGF9; 100 ng/ml of FGF9 and 0.18 U/ml (1 µg/ml) of heparin; 25 ng/ml of FGF9; 25 ng/ml of FGF9 and 0.18 U/ml (1 µg/ml) of heparin; 10 ng/ml of FGF9; 10 ng/ml of FGF9 and 0.18 U/ml (1 µg/ml) of heparin; or 0 ng/ml of FGF9, and also replacing the 100 ng/ml of FGF2 in step 4 of the differentiation induction method B (production of a metanephric-lineage late primitive streak: step 4) with 30 ng/ml of FGF2, hNPC were differentially induced from hiPSC using the same procedure as the differentiation induction method B.

For each of these differentially induced hNPC, the results of confirming the differentiation induction efficiency of SIX2-positive cells are illustrated in FIG.15. The percentages of SIX2-positive cells among the live cells not stained by DAPI are shown. The concentration of FGF9 in step 5 (production of a late posterior intermediate mesoderm: step 5) had no effect on the hNPC induction efficiency. The presence of heparin had no effect on the hNPC induction efficiency.

### (Example 8)

The hNPC were differentially induced from hiPSC in accordance with the protocol of the above differentiation induction method B.

Further, with the exceptions of using DMEM/F12 Glutamax supplemented with either 10% or 20% AS401 (StemFit (a registered trademark) for Differentiation, Ajinomoto Healthy Supply Co., Inc.) as the basal medium in steps 2 to 6 of the differentiation induction method B; replacing the 100 ng/ml of FGF2 in step 3 of the differentiation induction method B (production of a mesodermal-lineage late primitive streak: step 3) with 0 ng/ml of FGF2; replacing the 100 ng/ml of FGF 2 in step 4 of the differentiation induction method B (production of a metanephric-lineage late primitive streak: step 4) with 30 ng/ml of FGF2; replacing the 200 ng/ml of FGF9 in step 5 of the differentiation induction method B (production of a late posterior intermediate mesoderm: step 5) with 0 ng/ml of FGF9; and replacing the 200 ng/ml of FGF9 in step 6 of the differentiation induction method B (production of hNPC: step 6) with 100 ng/ml of FGF9 and 0.18 U/ml (1 µg/ml) of heparin, hNPC were differentially induced from hiPSC using the same procedure as the differentiation induction method B.

For each of these differentially induced hNPC, the results of confirming the differentiation induction efficiency of SIX2-positive cells are illustrated in FIG.16. In FIG. 16, the percentages of SIX2-positive cells among the live cells not stained by DAPI are shown. A tendency for improved induction efficiency of hNPC when using the basal medium supplemented with 10% AS401 was confirmed.

### (Example 9)

With the exception of using a clinical hiPSC stock line as the hiPSC, hNPC were differentially induced in the same manner as Example 8 (the differentiation induction method B). In this example, either B27, or DMEM/F12 Glutamax containing 10% added AS401 was used as the basal medium.

For each of these differentially induced hNPC, the results of ascertaining the SIX2 expression are illustrated in FIG.17. A tendency for improved hNPC induction efficiency was confirmed in the case where a basal medium containing 10% added AS401 was used.

### (Example 10)

Using the conditions illustrated in FIG. 18, hNPC were differentially induced from hiPSC (hereinafter referred to as "differentiation induction method C"). DMEM/F12 Glutamax containing 10% added AS401 was used as the basal medium. In the small scale procedure, a 24-well plate (Greiner Bio-One, 662160) was inoculated with iPS cells in stage 1, and following the first day of stage 4, the cells were re-inoculated to a 24-well plate. Following the second day of stage 6, the hNPC were collected, and a low-attachment 96-well plate (PrimeSurface plate 96U (Sumitomo Bakelite Co., Ltd., MS-9096U)) was used to conduct culturing and expansion in the CFY medium. In the large scale procedure, the iPS cells were inoculated into a T150 flask or T75 flask (Iwaki) in stage 1, and following the first day of stage 4, the cells were re-inoculated to a 512 cm² plate (Sumitomo Bakelite, adherent cell culture peel-off culture vessel 512, MS-28500). Following the second day of stage 6, the hNPC were collected, and a low-attachment 96-well plate was used to conduct culturing and expansion in the CFY medium.

FIG. 19 illustrates the results of confirming, by immunostaining, SIX2 expression of the hNPC induced from an HLA homostock hiPSC line (Ff14s04) using the small scale differentiation induction method (differentiation induction method C (Small)).

FIG. 20 shows cell morphological photographs and the level of SIX2 expression following implementation of either the small scale (differentiation induction method C (Small)) or large scale (differentiation induction method C (Large)) differentiation induction method illustrated in FIG. 18. The stage 6 morphological photographs and levels of SIX2 expression confirmed the differential induction of hNPC in both the small scale culture and the large scale culture. The morphological photographs on the seventh day of the culturing and expansion process confirmed that culturing and expansion using the CFY medium was possible using hNPC differentially induced from either the small scale or the large scale culture.

### (Example 11)

Using the differentiation induction method C (Large), hNPC were differentially induced from hiPSC. DMEM/F12 Glutamax containing 10% added AS401 was used as the basal medium.

The cell yield following stage 6 of the differentiation induction method C (Large) is shown in FIG. 21. In the method of this example, the production of hNPC at the 10⁸ scale was confirmed.

FIG. 22 shows a fluorescent image confirming, by immunostaining, SIX2 expression in the hNPC following stage 6.

### (Example 12)

In order to compare the effects of additives in the culturing and expansion process, a CFY medium containing 10% added AS401 instead of B27 (CFY medium (-B27, +10% AS401), a CFY medium containing 20% added AS401 instead of B27 (CFY medium (-B27, +20% AS401), and the CFY medium were prepared.

Using the differentiation induction method C (Small), hNPC were differentially induced from hiPSC. DMEM/F12 Glutamax containing 10% added AS401 was used as the basal medium. The hNPC were collected following stage 6, and a low-attachment 96-well plate was used to conduct culturing and expansion in the CFY medium, the CFY medium (-B27, +10% AS401), or the CFY medium (-B27, +20% AS401).

FIG. 23 illustrates morphological photographs of the hNPC during the culturing and expansion process. When the CFY medium or the CFY medium (-B27, +20% AS401) was used, the form of the hNPC mass changed to an oval shape. In contrast, when the CFY medium (-B27, +10% AS401) was used, the form of the hNPC mass was maintained as a circular shape.

FIG. 24 illustrates the growth rates. The hNPC exhibited the most favorable growth rate in the CFY medium (-B27, +10% AS401).

Based on these results, it is thought that the CFY medium (-B27, +10% AS401) is the most suitable for culturing and expanding hNPC.

### (Example 13)

Using the differentiation induction method C (Small), hNPC were differentially induced from hiPSC. DMEM/F12 Glutamax containing 10% added AS401 was used as the basal medium. The hNPC were collected following stage 6, and a low-attachment 96-well plate was used to conduct culturing and expansion in the CFY medium, the CFY medium (-B27, +10% AS401), or the CFY medium (-B27, +20% AS401).

The hNPC on the seventh day of culturing and expansion, equivalent to a cell mass of 3×10⁶ cells was transplanted beneath the renal capsule of an AKI mouse, and the therapeutic effect of the hNPC was ascertained.

The results of measuring the values for the blood urea nitrogen (BUN) and serum creatine (S-Cre) are shown in FIG. 25 and FIG. 26 respectively. A significant difference was not detected across the four groups including the control group, but a tendency for improvement in both the BUN and S-Cre values was observed in the transplant groups.

### (Example 14)

Using the differentiation induction method C (Small), hNPC were differentially induced from hiPSC. DMEM/F12 Glutamax containing 10% added AS401 was used as the basal medium. The hNPC were collected following stage 6, and a low-attachment 96-well plate was used to conduct culturing and expansion in either the CFY medium or the CFY medium (-B27, +10% AS401).

The results of confirming gene expression of the cell mass of hNPC on the seventh day of culturing and expansion are illustrated in FIG. 27. Culturing and expansion in the CFY medium (-B27, +10% AS401) tended to result in higher levels of expression for the NPC markers and an angiogenesis-related gene.

### (Example 15)

In order to ascertain the effect of FGF9 in the CFY medium, a medium was prepared by adding 300 ng/ml of FGF2 to the CFY medium instead of the FGF9 (CFY medium (-FGF9, +FGF2)).

Next, hNPC were differentially induced from hiPSC using the differentiation induction method A. DMEM/F12 Glutamax containing added B27 (B-27 Supplement, minus vitamin A, Invitrogen) was used as the basal medium. The hNPC were collected following stage 6, and a low-attachment 96-well plate was used to conduct culturing and expansion in either the CFY medium, or the CFY medium (-FGF9, +FGF2).

On the seventh day of culturing and expansion, the cell masses were inspected using an optical microscope and a fluorescence microscope, and the forms observed under the optical microscope and the NPC marker (OSR1, SIX2) expressions observed under the fluorescence microscope were compared. The results are shown in FIG. 28. Culturing and expansion in the medium containing FGF9 yielded higher expression of the NPC markers.

### (Example 16)

Renal organoids were differentially induced from hNPC that had been cultured and expanded using the CFY medium or the CFY medium (-FGF9, +FGF2).

The results are shown in FIG. 29. Culturing and expansion in the medium containing FGF9 yielded an increase in the surface area of the renal organoid (the light colored portion), indicating favorable formation of renal organoids.

### (Example 17)

In order to ascertain the effect of the ROCK inhibitor (Y-27632) in the CFY medium, a medium (CF medium) was prepared with the ROCK inhibitor excluded from the CFY medium.

Next, hNPC that had been maintained in the CFY medium was subcultured in the CFY medium. Two days after this passage, a medium replacement to either the CFY medium or the CF medium was performed (first medium replacement). Subsequently, two days after the first medium replacement, another medium replacement to either the CFY medium or the CF medium was performed (second medium replacement). Two days after this second medium replacement, a subculture was conducted in the CFY medium. Using this cycle, subculturing was conducted up until passage 3.

Cells were collected at the time of passage, and NPC marker-positive cells (OSR1, SIX2) were analyzed by flow cytometry.

The results are shown in FIG. 30 and FIG. 31. There were no effects on the expression of the NPC markers, regardless of whether the replacement medium used two days after passage was the CF medium (CFY → CF) or the CFY medium (CFY --+ CFY).

### (Example 18)

First, hNPC that had been maintained in the CFY medium was subcultured in the CFY medium. Two days after this passage, a medium replacement to either the CFY medium or the CF medium was performed (first medium replacement). Subsequently, after another two days, another medium replacement to either the CFY medium or the CF medium was performed (second medium replacement). Two days after this second medium replacement, a subculture was conducted in the CFY medium. Using this cycle, subculturing was conducted up until passage 3.

Cells were collected at the time of passage, and the proportions of cells positive for NPC markers (OSR1, SIX2) and cells positive for c-MET (a cell surface antigen protein specifically expressed in NPC) among the living cells not stained by DAPI were analyzed by flow cytometry.

The results are shown in FIG. 32 (passage 1) and FIG. 33 (passage 2). Cell numbers measured by flow cytometry and corresponding with the proportion of each cell group (OSR1(+)SIX2(+), OSR1(+)SIX2(-), c-MET(+)) within the total number of live cells not stained by DAPI are indicated in the figures. There were no effects on the expression of the NPC markers, regardless of whether the replacement medium used two days after passage was the CF medium (CFY → CF) or the CFY medium (CFY → CFY).

### (Example 19)

First, hNPC that had been maintained in the CFY medium was subcultured in the CFY medium. Two days after this passage, a medium replacement to either the CFY medium or the CF medium was performed (first medium replacement). Subsequently, after another two days, another medium replacement to either the CFY medium or the CF medium was performed (second medium replacement). Two days after this second medium replacement, a subculture was conducted in the CFY medium. Using this cycle, subculturing was conducted up until passage 2.

The cell masses following passage 2 were inspected using an optical microscope and a fluorescence microscope, and the forms observed under the optical microscope and the NPC marker (OSR1, SIX2) expressions observed under the fluorescence microscope were compared. The results are shown in FIG. 34. There were no effects on the expression of the NPC markers, regardless of whether the replacement medium used two days after passage was the CF medium (CFY → CF) or the CFY medium (CFY → CFY).

### (Example 20)

First, hNPC that had been maintained in the CFY medium was subcultured in the CFY medium. Two days after this passage, a medium replacement to either the CFY medium or the CF medium was performed (first medium replacement). Subsequently, after another two days, another medium replacement to either the CFY medium or the CF medium was performed (second medium replacement). Two days after this second medium replacement, a subculture was conducted in the CFY medium. Using this cycle, subculturing was conducted up until passage 1.

The hNPC following passage 1 was differentially induced into renal organoids. The morphology of the renal organoids was observed under an optical microscope. The results are shown in FIG. 35. Renal organoids were formed regardless of whether the replacement medium used two days after passage was the CF medium (CFY --> CF) or the CFY medium (CFY → CFY). Even when the replacement medium used two days after passage was the CF medium (CFY → CF), the renal organoid formation was similar to that observed when the CFY medium was used two days after passage (CFY --> CFY).

### [Industrial Applicability]

The present invention is able to provide a medium for culturing and expanding nephron progenitor cells that enables culturing and expansion of the nephron progenitor cells with good retention of the differentiation potency, a method for culturing and expanding NPC using the medium, and a method for producing renal organoids from nephron progenitor cells obtained using the culturing and expansion method. The nephron progenitor cells and renal organoids obtained using the methods of the present invention can be applied to the treatment or prevention of kidney disease.

## Claims

1. A medium for culturing and expanding nephron progenitor cells, the medium comprising a GSK-3β inhibitor, a ROCK inhibitor, and at least one fibroblast growth factor selected from the group consisting of FGF9 and FGF20.

2. The medium for culturing and expanding nephron progenitor cells according to Claim 1, further comprising a JAK inhibitor.

3. The medium according to Claim 2, wherein the JAK inhibitor is a JAK2 inhibitor.

4. The medium according to Claim 3, wherein the JAK2 inhibitor is TG101348.

5. The medium according to any one of Claims 1 to 4, wherein the GSK-3β inhibitor is CHIR99021.

6. The medium according to any one of Claims 1 to 5, wherein the ROCK inhibitor is Y-27632.

7. The medium according to any one of Claims 1 to 6, further comprising 10% by volume of AS401 relative to a total volume of the medium.

8. The medium according to any one of Claims 1 to 7, wherein the nephron progenitor cells are human nephron progenitor cells.

9. The medium according to any one of Claims 1 to 8, wherein the nephron progenitor cells are nephron progenitor cells induced from pluripotent stem cells.

10. The medium according to Claim 9, wherein the pluripotent stem cells are iPS cells.

11. A method for culturing and expanding nephron progenitor cells, the method comprising a step of culturing nephron progenitor cells in the medium according to any one of Claims 1 to 10.

12. The method for culturing and expanding nephron progenitor cells according to Claim 11, wherein the step of culturing nephron progenitor cells includes subculturing of the nephron progenitor cells.

13. A method for culturing and expanding nephron progenitor cells, the method comprising:
(A) a step of culturing nephron progenitor cells in the medium according to any one of Claims 1 to 10 for 30 to 60 hours, and
(B) a step of culturing the cells obtained in the step (A) in a medium comprising a GSK-3β inhibitor and at least one fibroblast growth factor selected from the group consisting of FGF9 and FGF20, but comprising no ROCK inhibitor.

14. The method for culturing and expanding nephron progenitor cells according to Claim 13, the method further comprising:
(C) a step of subculturing the cells obtained in the step (B) in the medium according to any one of Claims 1 to 10.

15. The method for culturing and expanding nephron progenitor cells according to Claim 14, the method further comprising:
(D) a step of culturing the cells subcultured in the step (C) in the medium according to any one of Claims 1 to 10 for 30 to 60 hours, and
(E) a step of culturing the cells obtained in the step (D) in a medium comprising a GSK-3β inhibitor and at least one fibroblast growth factor selected from the group consisting of FGF9 and FGF20, but comprising no ROCK inhibitor.

16. The method for culturing and expanding nephron progenitor cells according to any one of Claims 11 to 15, wherein the nephron progenitor cells are human nephron progenitor cells.

17. The method for culturing and expanding nephron progenitor cells according to any one of Claims 11 to 16, wherein the nephron progenitor cells are nephron progenitor cells induced from pluripotent stem cells.

18. The method for culturing and expanding nephron progenitor cells according to Claim 17, wherein the pluripotent stem cells are iPS cells.

19. The method for culturing and expanding nephron progenitor cells according to Claim 17 or 18, wherein the nephron progenitor cells are nephron progenitor cells obtained using a method comprising steps (i) to (vi) below:
(i) a step of culturing pluripotent stem cells in a medium comprising FGF2, BMP4, a GSK-3β inhibitor, and retinoic acid or a derivative thereof;
(ii) a step of culturing the cells obtained in the step (i) in a medium comprising FGF2, a GSK-3β inhibitor and BMP7;
(iii) a step of culturing the cells obtained in the step (ii) in a medium comprising a GSK-3β inhibitor, BMP7 and a TGFβ inhibitor, but comprising no FGF2;
(iv) a step of culturing the cells obtained in the step (iii) in a medium comprising FGF2, a GSK-3β inhibitor, activin and a ROCK inhibitor;
(v) a step of culturing the cells obtained in the step (iv) in a medium comprising retinoic acid or a derivative thereof, but comprising no FGF9; and
(vi) a step of culturing the cells obtained in the step (v) in a medium comprising a GSK-3β inhibitor and at least one fibroblast growth factor selected from the group consisting of FGF9 and FGF20.

20. The method for culturing and expanding nephron progenitor cells according to Claim 19, wherein the medium used in the step (iv) comprises no BMP7.

21. The method for culturing and expanding nephron progenitor cells according to Claim 19 or 20, wherein at least one of the steps (i) to (vi) is conducted using a culture vessel having a cell contact area of at least 400 cm².

22. A method for producing renal organoids, the method comprising:
a step of culturing and expanding nephron progenitor cells using the method for culturing and expanding nephron progenitor cells according to any one of Claims 11 to 21, and
a step of differentiating the cultured and expanded nephron progenitor cells into renal organoids.
